# EUROPEAN PATENT SPECIFICATION

(11) **EP 3 958 926 B1**
(45) Date of publication and mention of the grant of the patent: **20.08.2025**
(21) Application number: 20794229.3
(22) Date of filing: 27.04.2020
(51) Int. Cl.: A61F 2/06, A61L 27/50, A61L 27/52, B33Y 80/00, C12N 5/00, B33Y 10/00, B33Y 70/00

(54) **MULTIVASCULAR NETWORKS AND FUNCTIONAL INTRAVASCULAR TOPOLOGIES WITHIN BIOCOMPATIBLE HYDROGELS**
MULTIVASKULARE NETZWERKE UND FUNKTIONALE INTRAVASKULÄRE TOPOLOGIEN IN BIOKOMPATIBLEN HYDROGELEN
RÉSEAUX MULTIVASCULAIRES ET TOPOLOGIES INTRAVASCULAIRES FONCTIONNELLES DANS DES HYDROGELS BIOCOMPATIBLES

(30) Priority: 26.04.2019 US 201962839358 P
(43) Date of publication of application: 02.03.2022
(73) Proprietor: William Marsh Rice University, Houston, TX 77005 (US)
(72) Inventor: MILLER, Jordan, Houston, TX 77004 (US); GRIGORYAN, Bagrat, Houston, TX 77004 (US)
(74) Representative: Rückerl, Florian
(86) International application number: PCT/US2020/030153
(87) International publication number: WO 2020/220046

(56) References cited:
- WO-A1-2014/085809
- WO-A2-02/053193
- US-A1- 2009 004 272
- US-A1- 2009 187 083
- US-A1- 2018 002 658
- US-B2- 9 232 805
- ZHANG RUJING ET AL: "Stereolithographic hydrogel printing of 3D culture chips with biofunctionalized complex 3D perfusion networks", LAB ON A CHIP, vol. 17, no. 24, 1 January 2017 (2017-01-01), UK, pages 4273 - 4282, XP055775953, ISSN: 1473-0197, Retrieved from the Internet <URL:https://pubs.rsc.org/en/content/articlepdf/2017/lc/c7lc00926g> DOI: 10.1039/C7LC00926G
- LUIZ E. BERTASSONI ET AL: "Hydrogel bioprinted microchannel networks for vascularization of tissue engineering constructs", LAB ON A CHIP, vol. 14, no. 13, 1 January 2014 (2014-01-01), UK, pages 2202 - 2211, XP055562319, ISSN: 1473-0197, DOI: 10.1039/C4LC00030G

## Description

### TECHNICAL FIELD

This disclosure relates to the formation of multivascular networks and functional intravascular topologies within biocompatible hydrogels.

### BACKGROUND

Solid organs transport fluids through distinct vascular networks that are biophysically and biochemically entangled, creating complex three-dimensional (3D) transport regimes that are difficult to produce and study. The morphologies of the circulatory and pulmonary systems are physically and evolutionarily entangled. In air-breathing vertebrates, these bounded and conserved vessel topologies interact to enable the oxygen-dependent respiration of the entire organism.

US 2018/002658 describes a hydrogel matrix comprising a plurality of layers, each layer comprising a cross-linked polymer network formed from a photosensitive polymer; a first elongated void in the hydrogel matrix providing a first tubular channel; a second elongated void in the hydrogel matrix providing a second tubular channel; wherein the first tubular channel and the second tubular channel are perfusable; wherein the first tubular channel does not intersect the second tubular channel; and wherein the second tubular channel interpenetrates the first tubular channel.

### SUMMARY

The present invention is defined by the independent claims, while the dependent claims concern the preferred embodiments. Any "embodiment" or "example" which is disclosed in the description but is not covered by the claims should be considered as presented for illustrative purpose only. The following detailed description describes the preparation of monolithic transparent hydrogels by stereolithographic production. The cytocompatible hydrogels contain intricate and functional vascular architectures, and have functional vascular topologies for studies of fluid mixers, valves, intervascular transport, nutrient delivery, and host engraftment. These intravascular and multivascular designs are created with photopolymerizable hydrogels by using food dye additives as biocompatible photoabsorbers for the projection stereolithography. The stereolithographic process enables simultaneous and orthogonal control over tissue architecture and biomaterials for the design of regenerative tissues. The entangled vascular networks formed from space-filling mathematical topologies enable creation of complex geometries.

According to the claimed invention, a device has a photopolymerizable hydrogel matrix including a photoabsorber and a void architecture in the matrix, having a first vessel architecture and a second vessel architecture that are each tubular and branching, where the first and second vessel architectures are fluidically independent from each other, and wherein one or both of the first vessel architecture and second vessel architecture is formed from a model based on a tessellation of polyhedra.

Implementations can include one or more of the following. The device allows at least 90% of visible light incident on the device to pass through and allow imaging of the device. The photoabsorber has been at least partially washed out of the device. The photoabsorber is degradable independent of any degradation of the remaining hydrogel matrix. The photoabsorber is degradable by chemical or physical processes. The photoabsorber is photobleachable, or removable by boiling. The model is based on a tessellation of polyhedra represents blood vessels. The model is based on a tessellation of polyhedra represents an airway. In various embodiments, the multi-vascular void architecture has a feature of a functional valve. In various embodiments, the valve is a bicuspid valve, tricuspid valve, monocuspid valve, or Tesla valve. In various embodiments, the multi-vascular void architecture has a feature of a fluidic static mixer. In various embodiments, the fluidic static mixer has between two and fifty fin elements. The feature is positionable at any position in the multi-vascular void architecture. The device is monolithic. The hydrogel matrix is biodegradable. The device is produced by additive manufacturing. The first vessel architecture and the second vessel architecture are entangled. The first vessel architecture is ensheathed by the second vessel architecture. The hydrogel matrix is porous. The device is implantable. The photoabsorber is hydrophilic. The photoabsorber is one of a food dye, tartrazine, Sunset Yellow FCF (Yellow No. 6), Brilliant Blue FCF (FD&C Blue No. 1), indigo carmine (FD&C Blue No. 2), Fast Green FCF (FD&C Green No. 3) anthocyanins, anthocyanidin, erythrosine (FD&C Red No.3), Allura Red AC (FD&C Red No. 40), riboflavin (Vitamin B2, E101, E101a, E106), ascorbic acid (vitamin C), Quinoline Yellow WS, carmoisine (azorubine), Ponceau 4R (E124), Patent Blue V (E131), Green S (E142), Yellow 2G (E107), Orange GGN (E111), Red 2G (E128), caramel color, phenol red, methyl orange, 4-nitrophenol, and NADH disodium salt. The photoabsorber is hydrophobic. The photoabsorber is one of curcumin (E100), turmeric, alpha carotene, beta carotene, canthaxanthin (keto-carotenoid), cochineal extract, paprika, saffron, ergocalciferol (vitamin D2), cholecalciferol (vitamin D3), Citrus Red 2, annatto extract, and Lycopene. Three or more vessel architectures fluidically independent from each other and from the first and second vessel architectures.

In some embodiments, a pre-polymerization solution has a photosensitive polymer, and a biocompatible, light-absorbing additive material suitable to control light penetration, where the additive material is at least partially removable from a solid formed of the pre-polymerization solution. The solution include plant, bacterial, or mammalian cells.

A device according to the claimed invention can be formed by a method of fabricating a 3D hydrogel construct by creating a 3D model of the construct based on a tessellation of polyhedra having a number of faces connected by edges and vertices. Subsequently generating a vascular component of the model by removing the faces and optionally the vertices of the polyhedra, and using the remaining topology as a vascular skeleton, and skinning the skeleton with a polygonal mesh and then smoothing the result to produce cylindrical channels along the edges of the model with intervessel junctions located at each vertex location. Then generating an airway component of the model by scaling the faces of the 3D model along local face normals such that the airway is nested inside the vasculature (vascular) component, forming independent fluidic connections to the vasculature and to the airway, and performing a Boolean subtraction from a solid volume, and combining the vascular component and the airway component of the model.

In some embodiments, fabricating a hydrogel alveolar construct includes fusing multiple spheres in a radially symmetric fashion to create an airway topology, offsetting the airway topology to generate a vascular surface topology, tessellating, skelatonizing, and skinning the vascular surface topology to produce a Voronoi vascular topology, where tessellating comprises performing a tessellation of polyhedra, and performing a Boolean subtraction of the original airway and the tessellated Voronoi vascular topology from a solid volume to generate a final 3D model for additive manufacturing. The tessellation of polyhedra is a Weaire-Phelan space-filling foam model of two dodecahedron and six tetrakaidecahedron cells.

In some embodiments, a method of manufacturing a hydrogel matrix construct includes creating a 3D model of the hydrogel matrix construct using a design software, where the 3D model of the hydrogel matrix construct comprises a first computational algorithm that yields a first tubular channel network in the hydrogel matrix construct, and a second computational algorithm, different from the first computational algorithm, that yields a second tubular channel network in the hydrogel matrix, where the first and second tubular channel networks are two independent, entangled vascular networks. Then by converting the 3D model to a format suitable for use in a 3D additive manufacturing software to yield a formatted 3D model to be generated using a 3D additive manufacturing machine, and directing the additive manufacturing machine to generate the model.

Implementations can include one or more of the following. The computational algorithm is a tessellation of polyhedra. Supplying a pre-polymerization solution to the 3D additive manufacturing machine where the pre-polymerization solution includes a photoabsorber. The pre-polymerization solution comprises one or more bacterial, mammalian, and plant cells. Lining the first tubular channel network or second tubular channel network with cells. Embedding the hydrogel matrix with cells. The cells are one or more of liver, lung, bone, endothelial, cardiac, pancreas, kidney, epithelial, muscle, cartilage, stem cells, skin, or eye cells. Delivering heat while the 3D additive manufacturing machine generates the 3D model. Heat is added via a silicone heater, heat lamps, or infrared LEDs. Enclosing the 3D model in a heating enclosure during generation of the 3D model.

Also described herein, but not forming part of the claimed invention, is a method of treating a subject in need thereof comprising implanting any of the devices into the subject, and a device comprising multiple joined subunits, where each subunit is any of the devices.

Advantages of the methods and materials of this description include use of stereolithography to efficiently convert photoactive liquid resins into structured plastic parts through localized photopolymerization. Compared to extrusion 3D printing, which deposits voxels in a serial fashion, photocrosslinking can be highly parallelized via image projection to simultaneously and independently address millions of voxels per time step. In stereolithography, xy-resolution is determined by the light path while z-resolution is dictated by light attenuating additives that absorb excess light and confine the polymerization to the desired layer thickness, thereby improving pattern fidelity. In the absence of suitable photoabsorber additives, 3D photopatterning of soft hydrogels can be been limited in the types of patterns that can be generated, or has implemented complex, expensive, and low-throughput microscopy. Common light blocking chemicals utilized for photoresist patterning or plastic part fabrication are not suitable for biomanufacturing due to their known genotoxic and carcinogenic characteristics. The use of non-toxic light blockers for projection stereolithography provides a major advance to the architectural richness available for the design and generation of biocompatible hydrogels.

### DESCRIPTION OF DRAWINGS

The accompanying drawings are not intended to be drawn to scale. Like reference numbers and designations in the various drawings indicate like elements. For purposes of clarity, not every component may be labeled in every drawing. In the drawings:
FIGS. 1A-H illustrate monolithic hydrogels with a functional intravascular topology in the form of a static mixer.
FIG. 2A illustrates monolithic hydrogels with a functional intravascular topology in the form of a bicuspid valve.
FIG. 2B show compliance measurement results for monolithic hydrogels with a functional intravascular topology in the form a horizontal channel.
FIGS. 3A-G illustrate adaptations of mathematical space-filling curves into entangled vessel topologies within hydrogels.
FIGS. 3H-J are results showing perfusion of deoxygenated RBCs through a vascular network such as in FIG. 3E.
FIGS. 4A-G illustrate engraftment of functional hepatic hydrogel carriers in an animal model.
FIGS. 5A-G illustrate details of the tessellation of polyhedra to generate 3D models for additive manufacturing of bioinspired alveoli hydrogels.
FIGS. 6A-H illustrate the use of 3D space-filling tessellations of polyhedra to replicate an ensheathing vasculature that mimics airway topography.

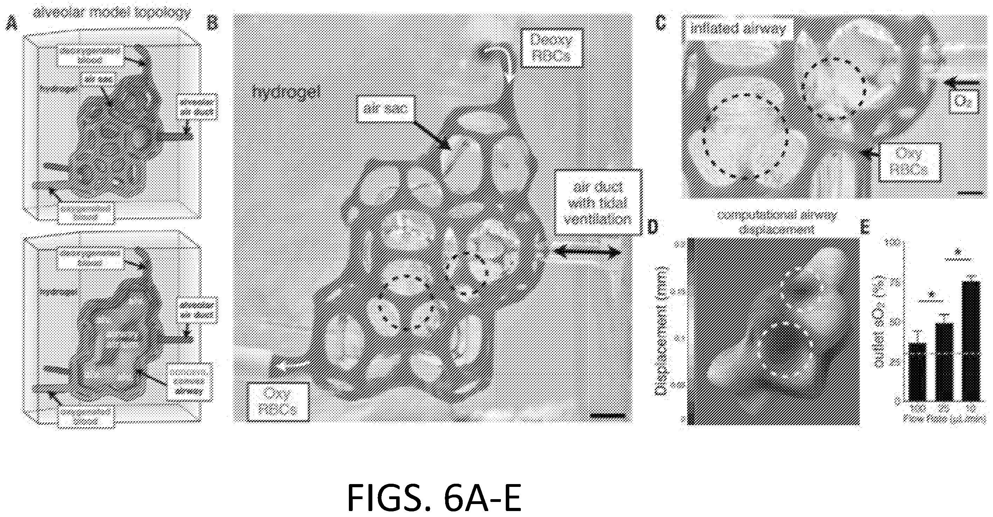

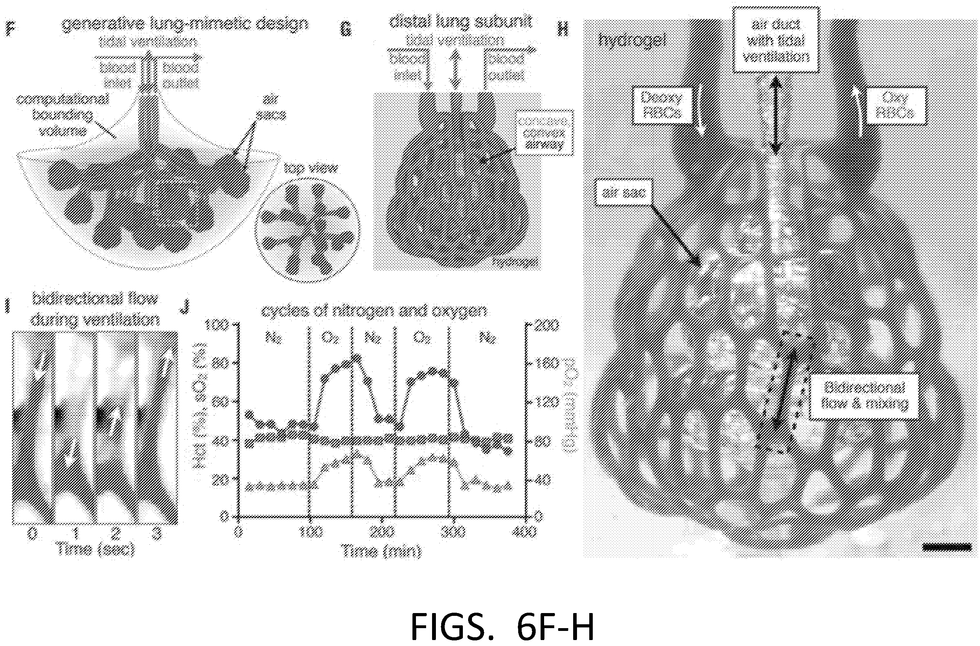

FIGS. 6A-I illustrate a bioinspired alveolar model with an ensheathing vasculature from 3D tessellations of Weaire-Phelan topology.

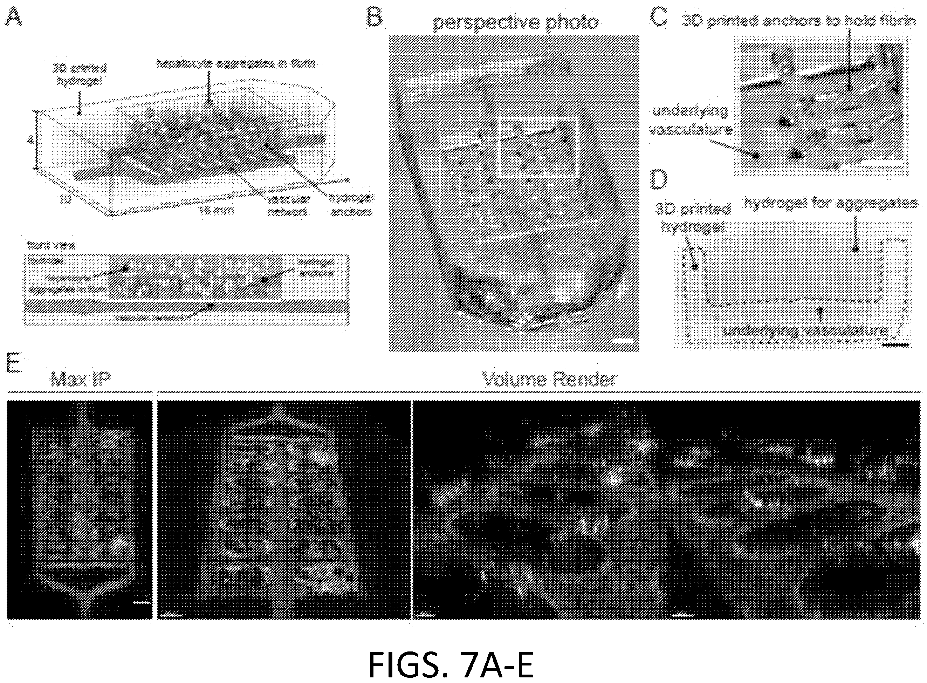

FIGS. 7A-E show additional imaging and development of hepatic hydrogel carriers.

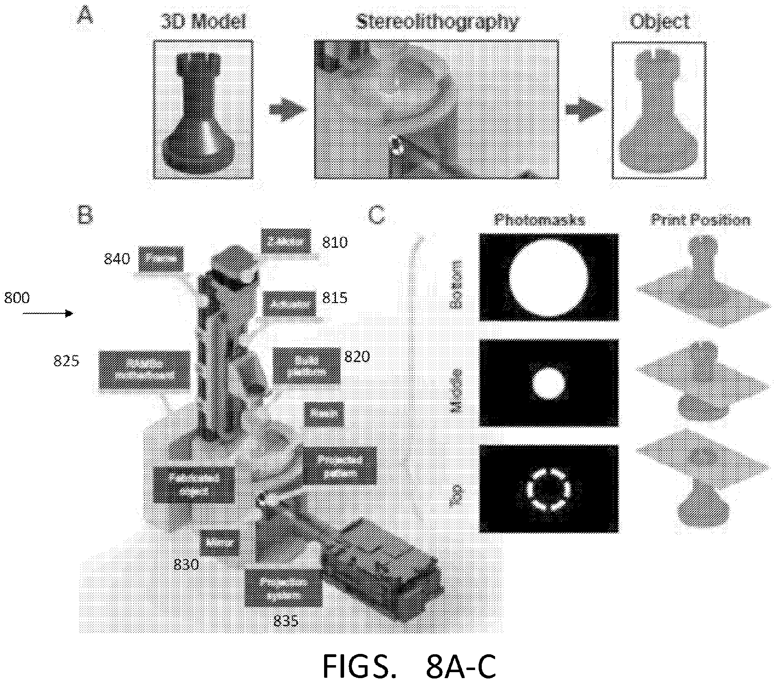

FIGS. 8A-C show the parts and steps for Stereolithography Apparatus for Tissue Engineering (SLATE).

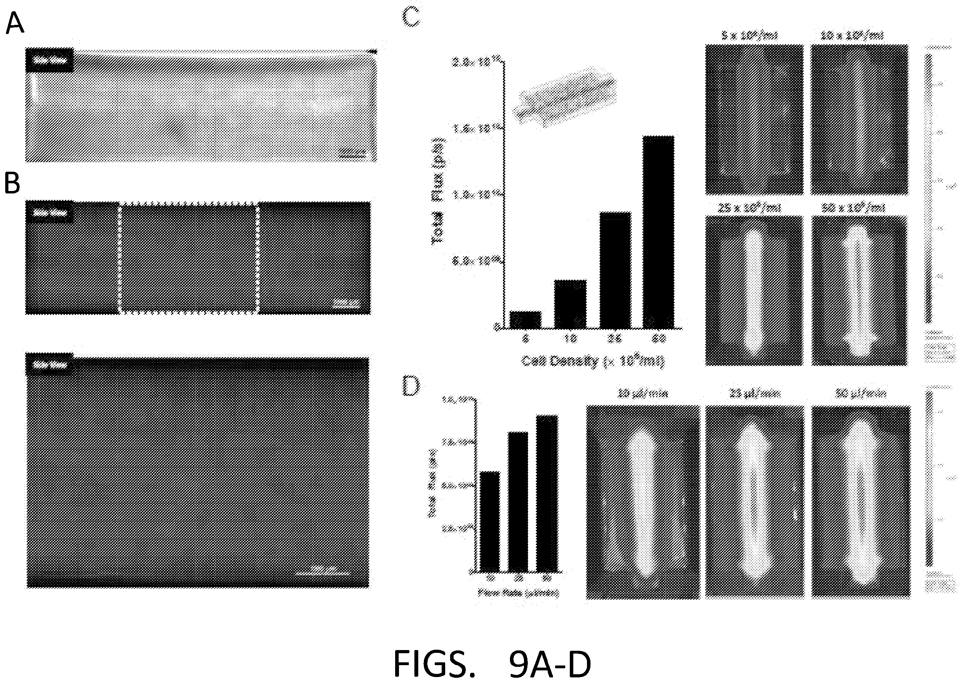

FIGS. 9A-D are experimental results indicating that adding xanthan gum in the pre-hydrogel solution results in homogeneous distribution of cells.

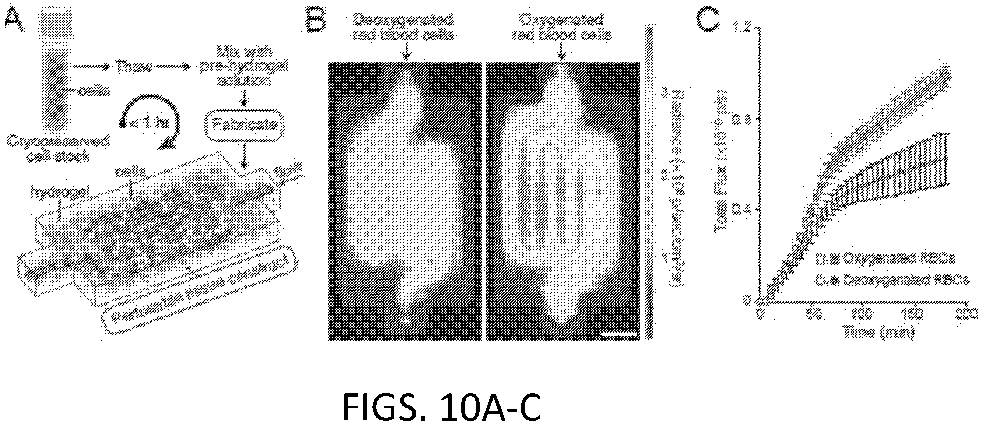

FIGS. 10A-C illustrate rapid biomanufacturing with cryopreserved cell stocks and non-invasive characterization of cellular activity.

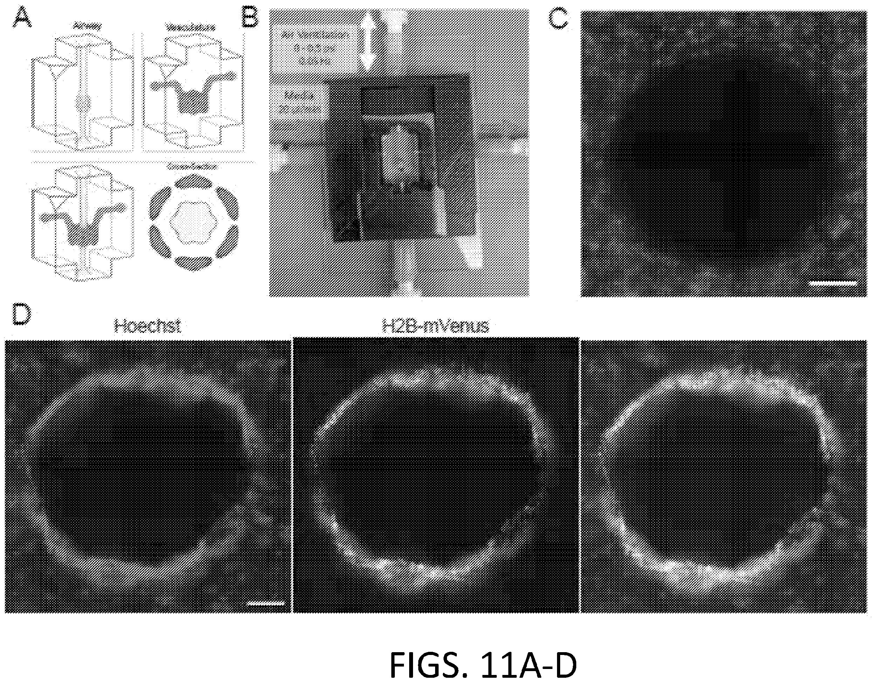

FIGS. 11A-D illustrate a monolithic lung-mimetic perfusion and ventilation tissue culture system.

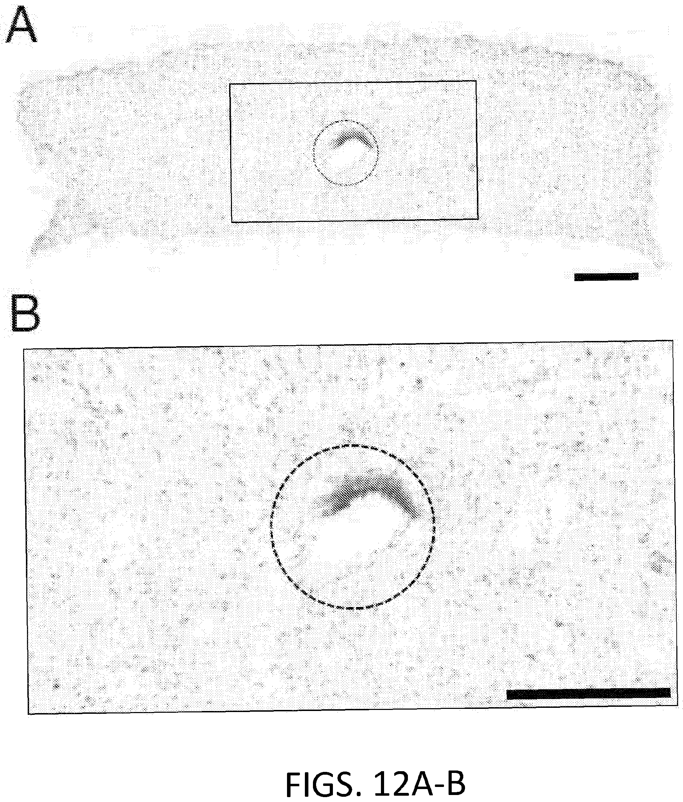

FIGS. 12A and 12B are stained sections showing hMSC differentiation of perfused single channel gels.

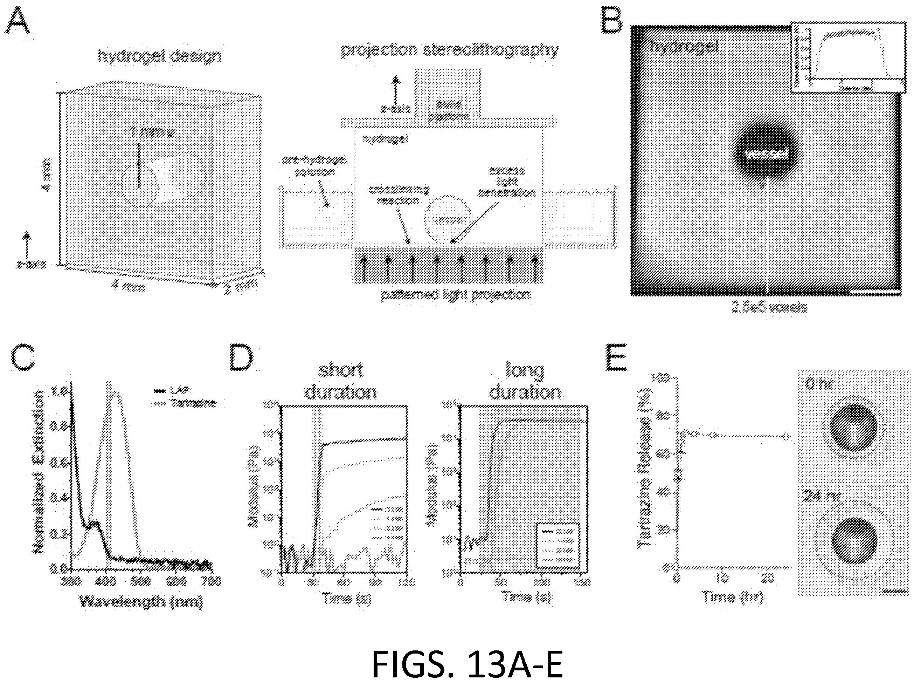

FIGS. 13A-E illustrate the fabrication of patent vessels within monolithic hydrogels.

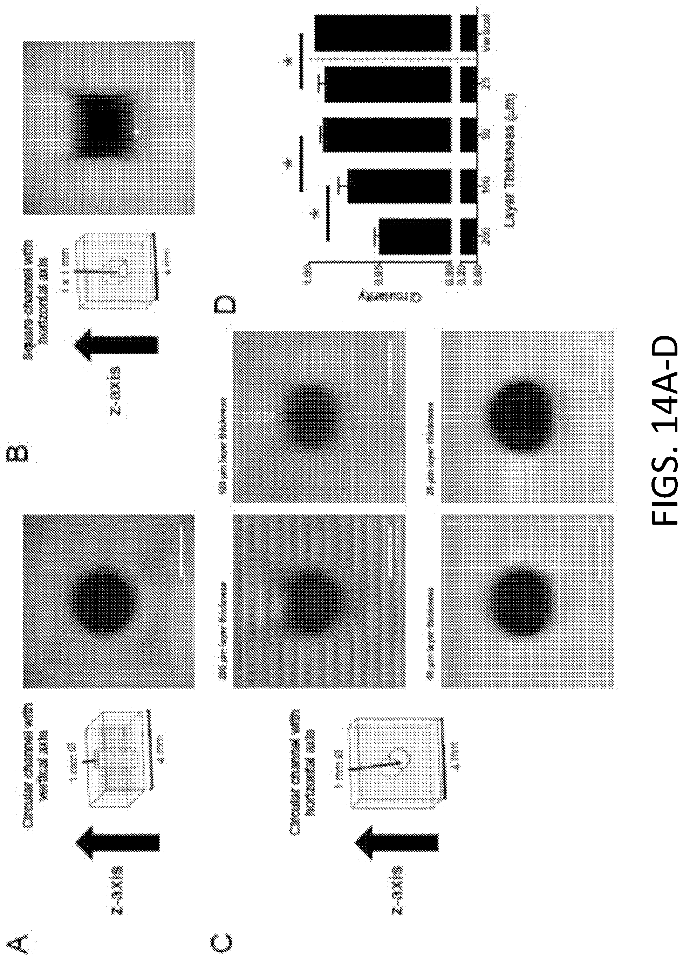

FIGS. 14A-D show experimental results indicating that fabrication orientation and layer thickness affect the fidelity of printed channels.

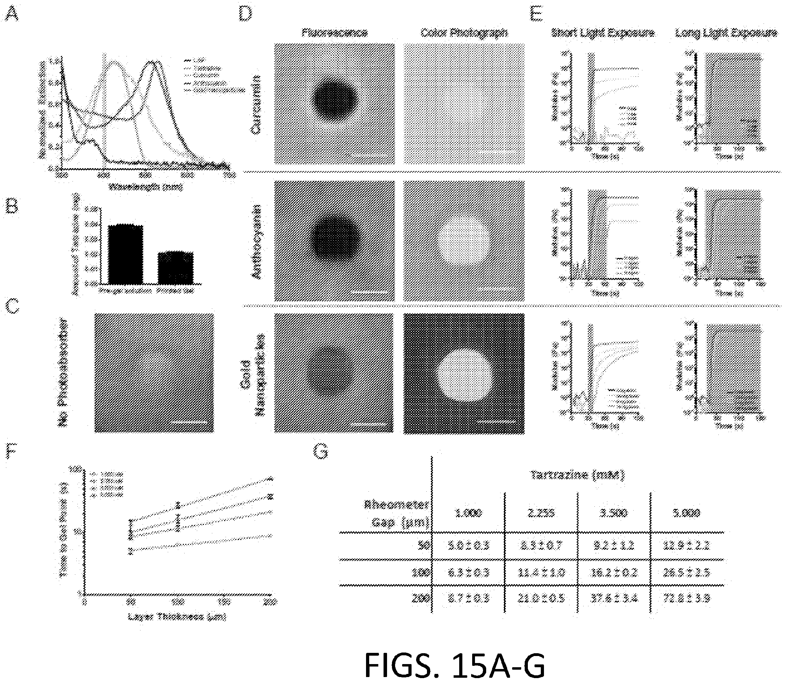

FIGS. 15A-G are experimental results indicating that biocompatible photoabsorbers allow fabrication of hydrogels with open channels.

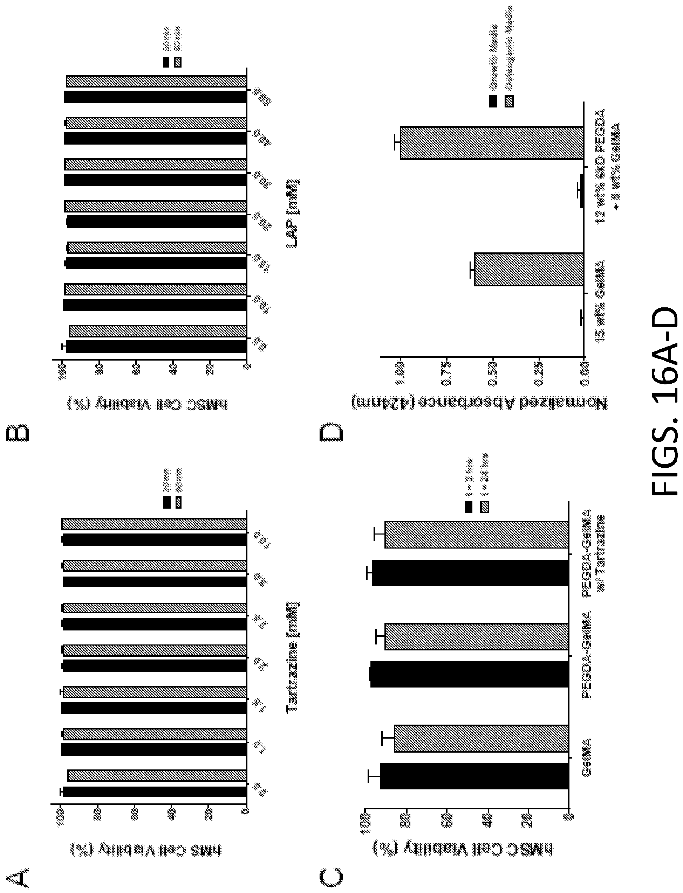

FIGS. 16A-D are experimental results indicating that the inclusion of tartrazine does not affect viability and differentiation of human mesenchymal stem cells.

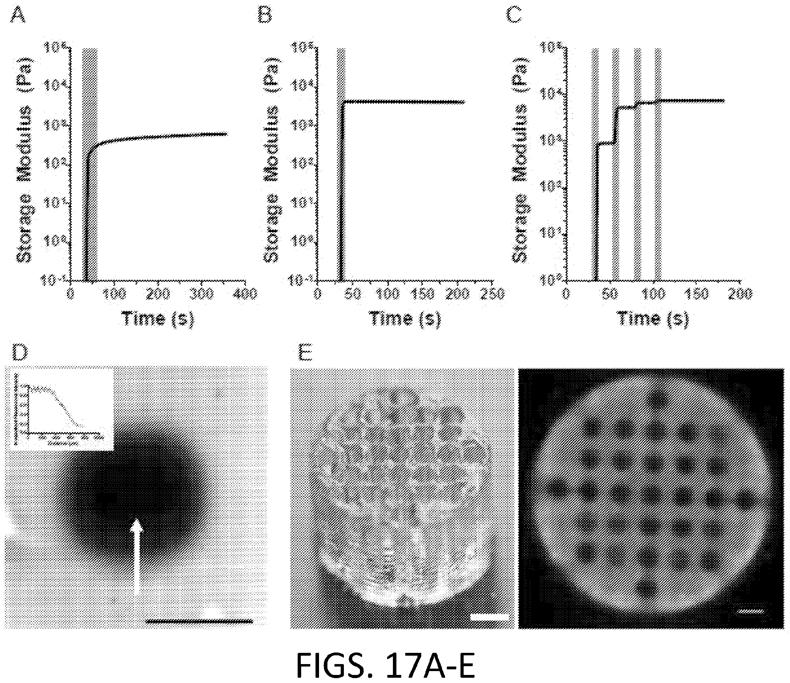

FIGS. 17A-E are results illustrating the compatibility of photoabsorber additives with advanced materials and fabrication strategies.

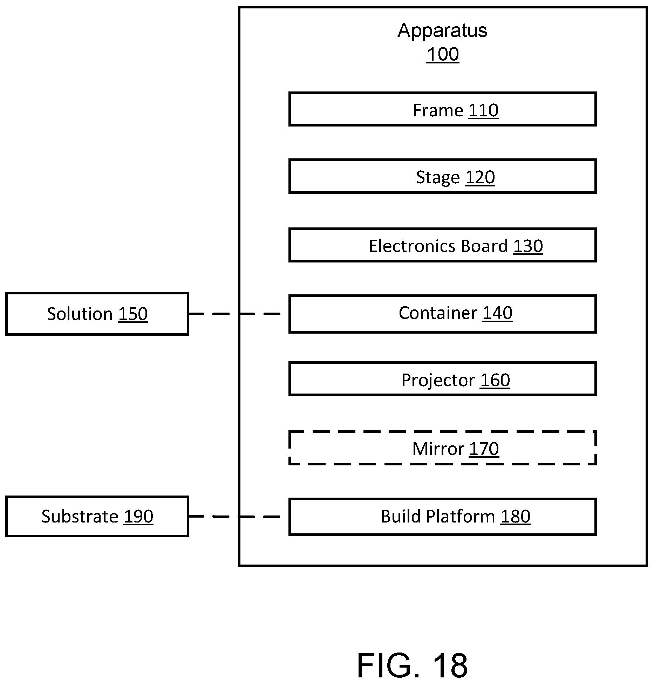

FIG. 18 is a schematic illustration of an example apparatus for fabricating a hydrogel construct, in accordance with various embodiments.

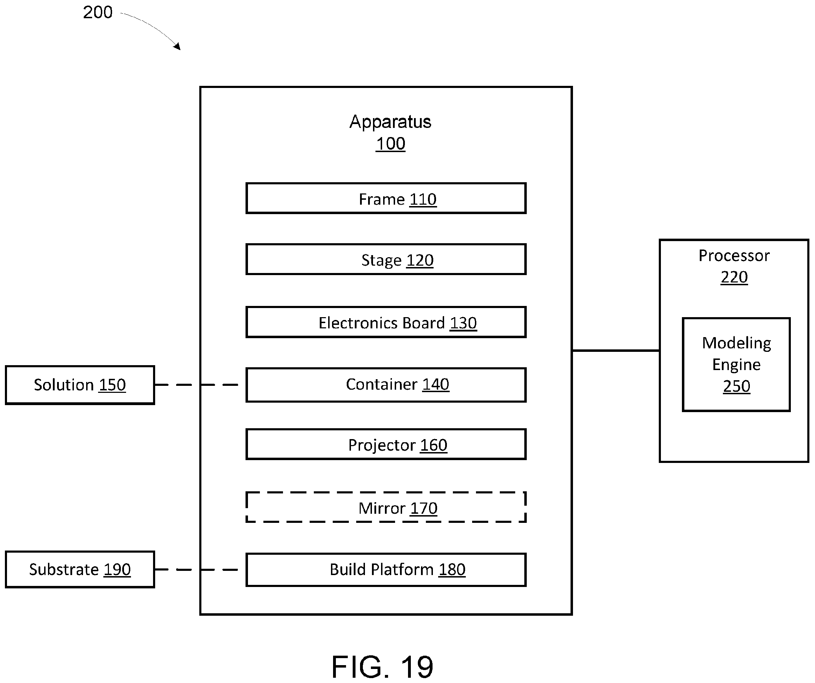

FIG. 19 is a schematic illustration of a system for manufacturing a hydrogel construct, in accordance with various embodiments.

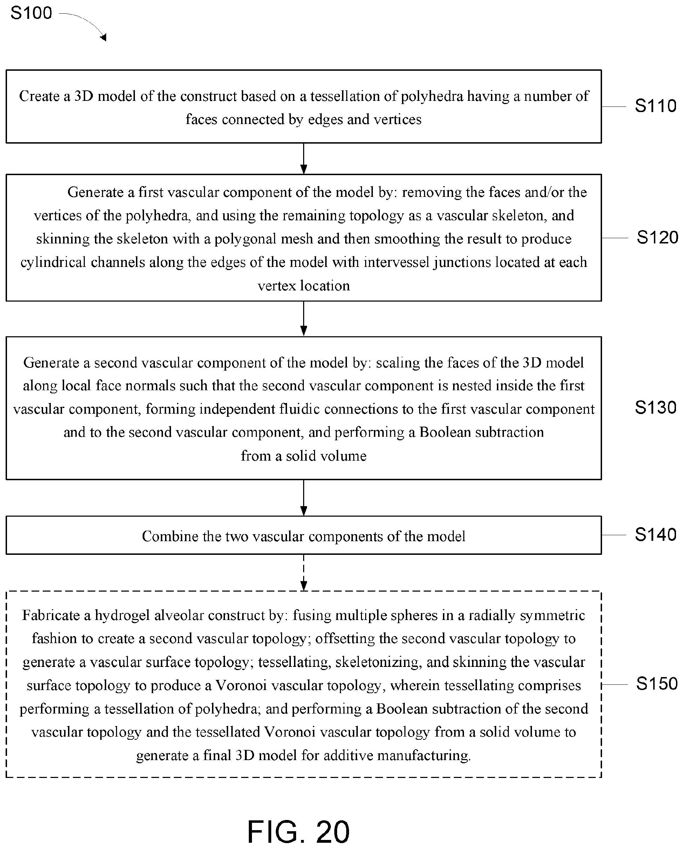

FIG. 20 is a flowchart for an example method of fabricating a 3D hydrogel construct, in accordance with various embodiments.

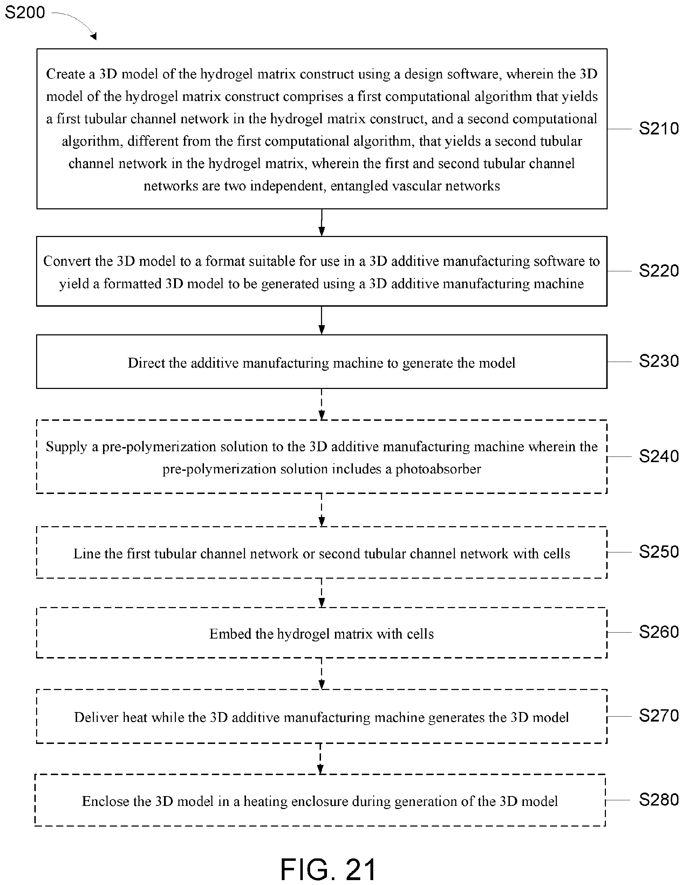

FIG. 21 is a flowchart for an example method of manufacturing a hydrogel matrix construct, in accordance with various embodiments.

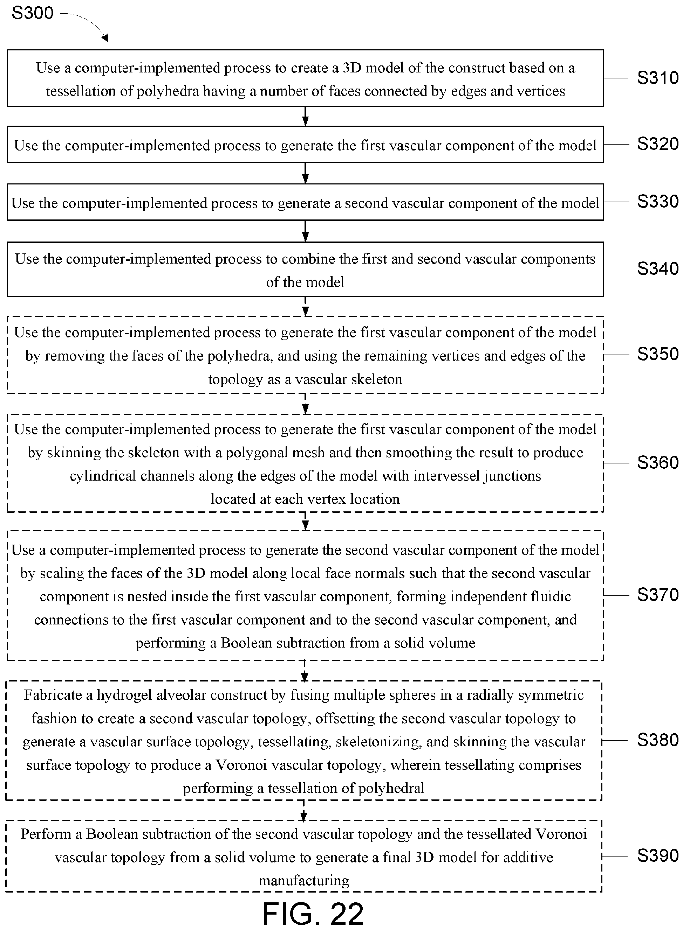

FIG. 22 is a flowchart for another example method of fabricating a 3D hydrogel construct, according to various embodiments.

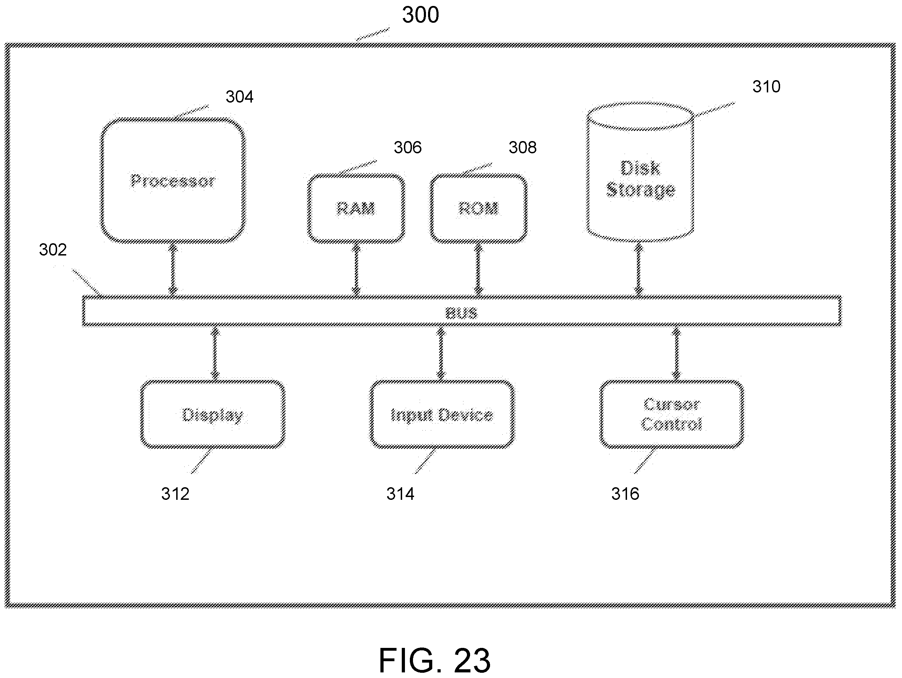

FIG. 23 is a block diagram that illustrates a computer system, in accordance with various embodiments.

It is to be understood that the figures are not necessarily drawn to scale, nor are the objects in the figures necessarily drawn to scale in relationship to one another. The figures are depictions that are intended to bring clarity and understanding to various embodiments of apparatuses, systems, and methods disclosed herein. Wherever possible, the same reference numbers will be used throughout the drawings to refer to the same or like parts. Moreover, it should be appreciated that the drawings are not intended to limit the scope of the present teachings in any way.

### DETAILED DESCRIPTION

Devices can be formed of a photopolymerizable hydrogel that includes a photoabsorber. During fabrication, the photoabsorber can be removed from the device, whether by being washed out, boiled out, or is degradable by another physical or chemical process that allows the photoabsorber to degrade independent of any other constituents in the hydrogel matrix. The resulting biocompatible device is biodegradable and porous, and suitable for implant into a patient, or for scientific studies.

The devices are formed to include complex internal structures while still being monolithic. These internal structures form a void architecture in the hydrogel matrix that takes the form of first and second vessel architectures that are each tubular and branching, but fluidically independent from each other, e.g., a multi-vascular void architecture. The first and second vessel architectures can be complex, entangled, or the first vessel architecture ensheathed by the second vessel architecture. The multi-vascular void architecture can take many forms, whereby one or both of the first and second vessel architectures is formed from a model based on a tessellation of polyhedra that can represent blood vessels or an airway.

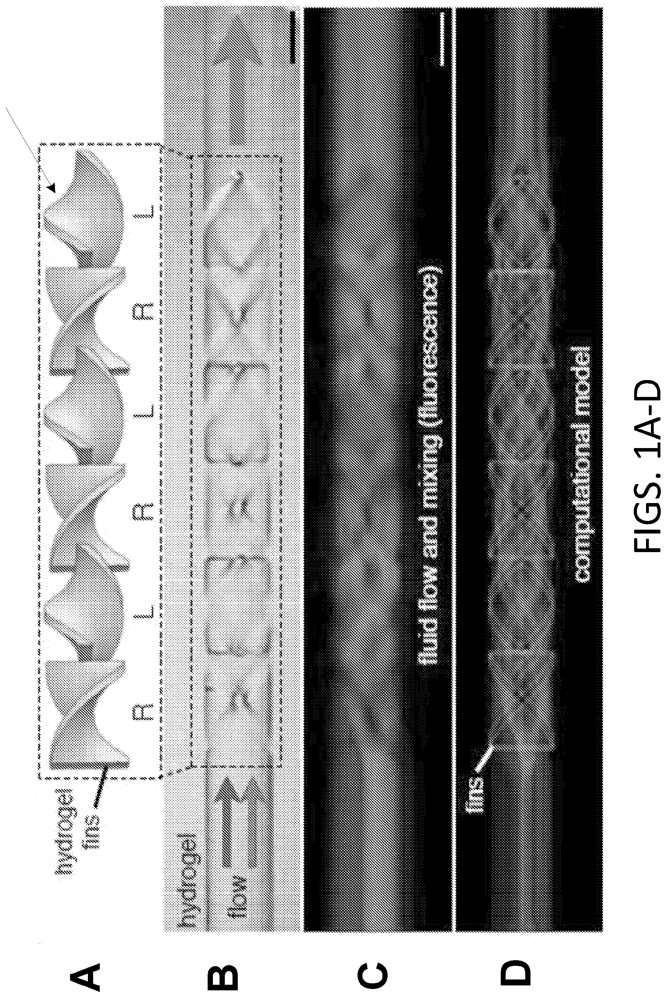

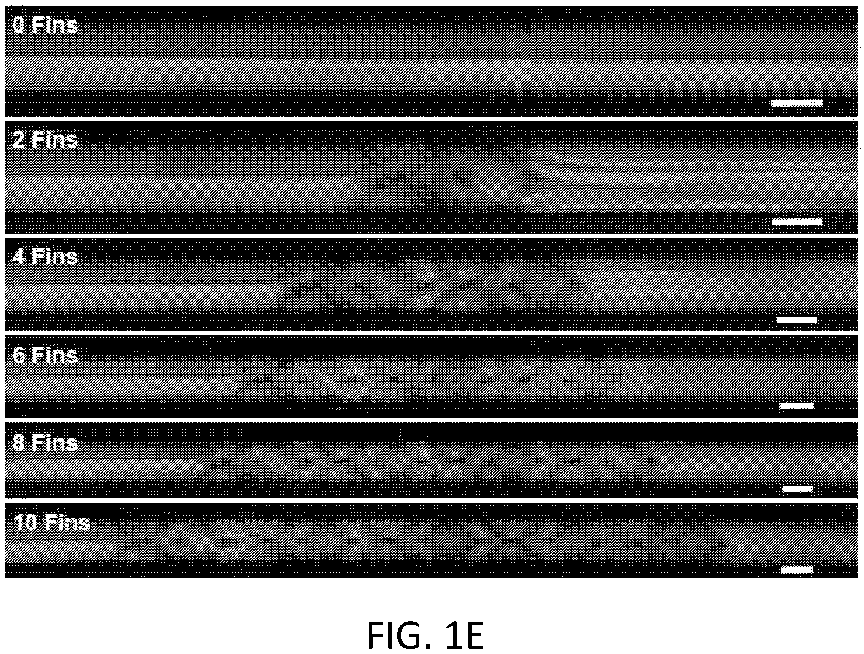

FIGS. 1A-H describe the validation of monolithic hydrogels generated with a simple functional intravascular topology, in this case a static mixer with fins that homogenizes fluid flows entering the mixer. FIG. 1A is a schematic of a monolithic hydrogel's interior integrated static mixer composed of 3D twisted fin elements of alternating chirality. FIG. 1B pictures a fabricated hydrogel having the integrated fin element configuration of FIG. 1A that is 150 µm thick inside a 1 mm cylindrical channel. Two fluid streams incident on the channel (for example, laminar fluid streams at a low Reynolds number (e.g., Re = 0.002)) are intermixed by interaction with the fins, as illustrated in the fluorescent image of FIG. 1C. The fluorescence imaging of FIG. 1C matches a computational model of flow (FIG. 1D). The laminar fluids' interaction with the static mixer varies with fin number, as shown in FIG. 1E's stitched fluorescence images of printed monolithic hydrogels (80 wt% 6 kDa) containing static mixers with 2 to 10 fin elements and perfused with fluorescent dyes.

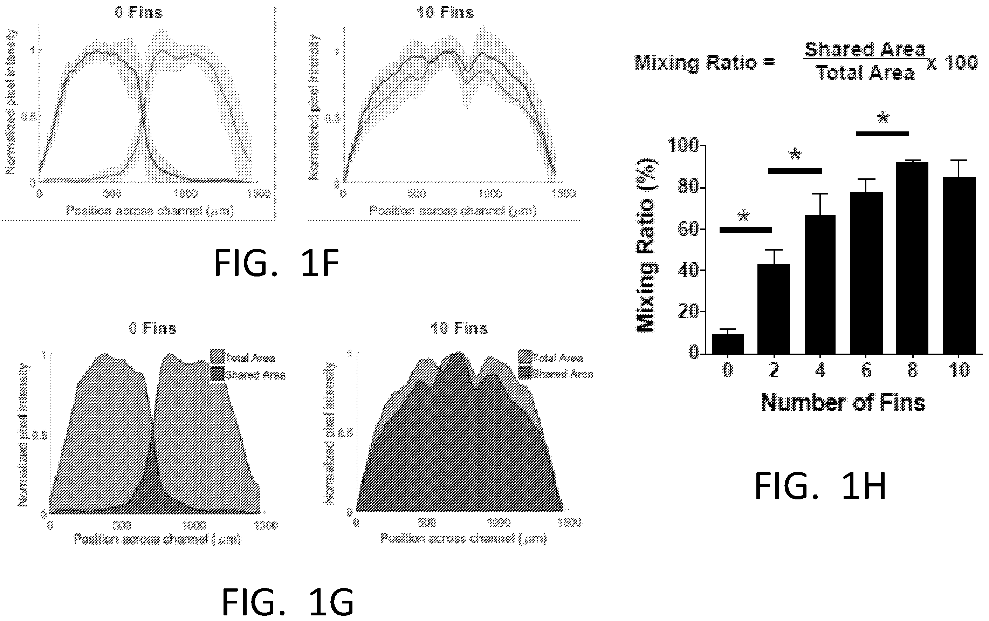

FIGS. 1F-H further quantifies the mixing efficiency of perfusable static mixers formed from the monolithic hydrogels described herein. FIG. 1F shows the normalized fluorescence intensity profiles at the outlet of a static mixer with 0 or 10 fins (the solid line represents the average intensity over a 3 mm length of the outlet immediately following the fins and the shaded regions represent standard deviation). FIG. 1G shows the average intensity data from FIG. 1F replotted to demonstrate mixing ratio calculations for static mixers. FIG. 1H shows a plot of mean mixing ratio by number of fins. These data illustrate that the fabricated hydrogel devices with their intravascular topology have predictable functionality.

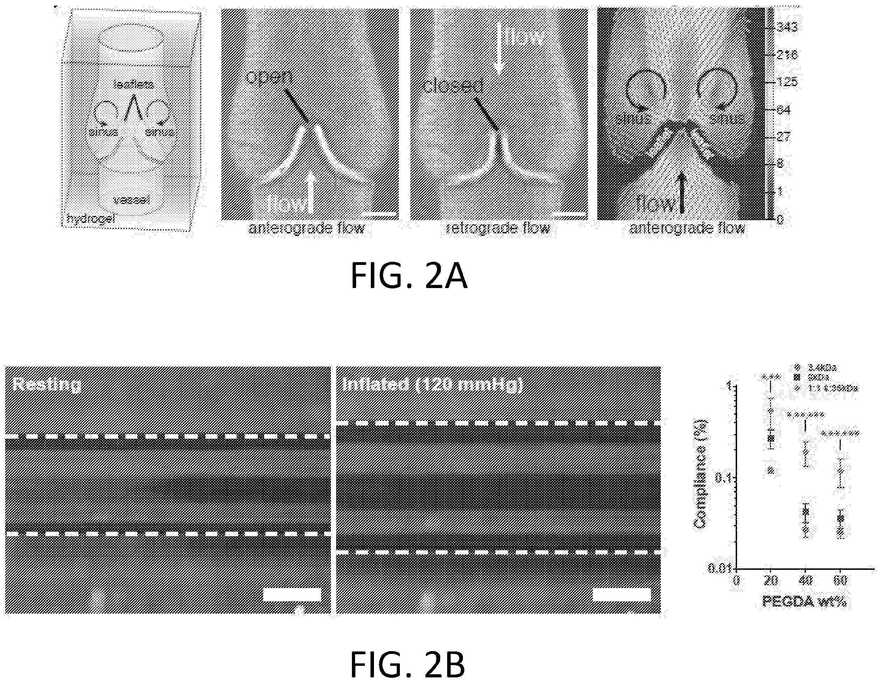

FIG. 2A-B illustrates the use of the fabricated hydrogel devices in the form of a 3D functional bicuspid venous valve. The valve leaflets are dynamic, rapidly respond to pulsatile anterograde and retrograde flows, and promote the formation of stable mirror image vortices in the valve sinuses according to established mappings of native tissue. The Particle Image Velocimetry (PIV) image at the right of FIG. 2A demonstrates stable mirror image vortices in the sinus region behind open valve leaflets.

FIG. 2B shows example compliance measurement results of horizontal channels fabricated within the hydrogels. At the left and center, vessel distension was measured upon application of pneumatic air pressure (hydrogel composition: 20 wt% 6 kDa PEGDA; channel diameter: 1.5 mm; channel length: 20 mm) both at rest and inflated to 120 mmHg. White dashed lines represent walls of the imaged channel. On the right, the graphed data indicates that compliance of vessels was found to be dependent on composition of pre-hydrogel solution. Solutions composed of lower polymer molecular weight and higher wt% resulted in hydrogels with lower compliance. The data of FIGS. 2A and 2B illustrate the viability of the hydrogel devices for use in multivascular systems.

Solid organs contain distinct fluid networks that are physically and chemically entangled. Separate vascular networks generally do not make a direct fluid connection to prevent being topologically reduced to a single connected network. Multivascular topologies within biocompatible and aqueous environments enables design of entangled networks that are suitable blueprints for fabrication within the hydrogels.

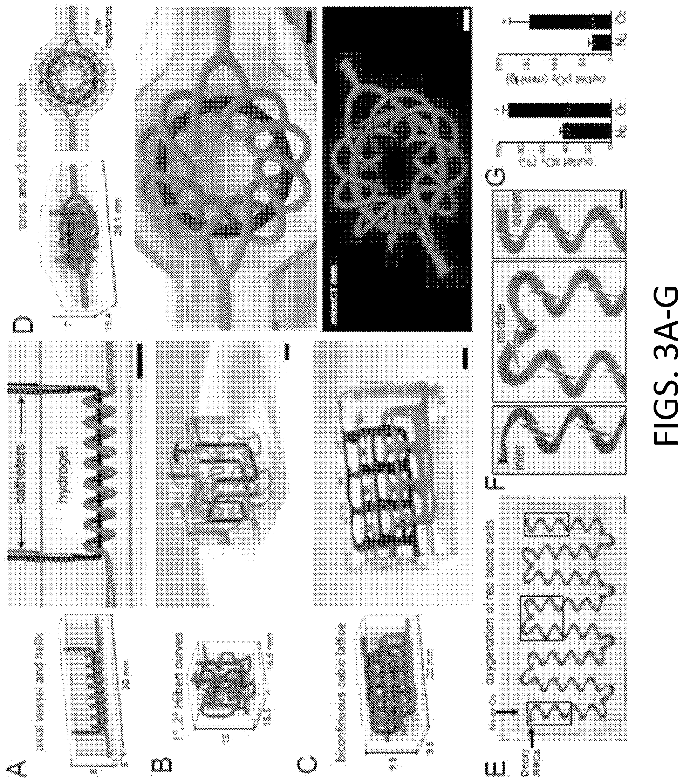

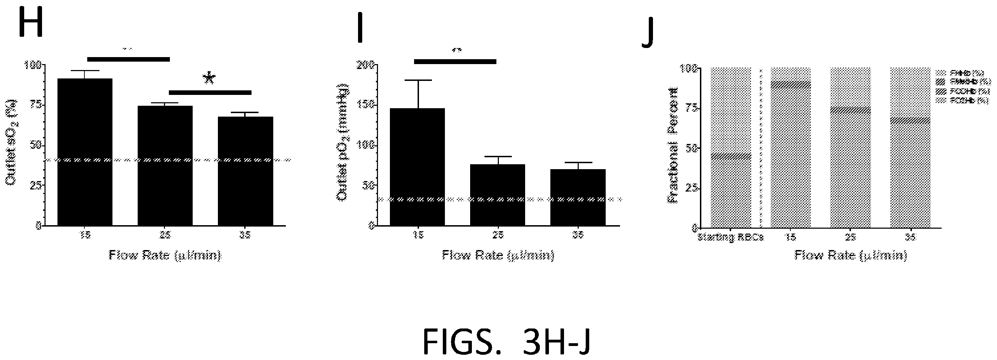

Mathematical space-filling and fractal topology algorithms provide an efficient parametric language to design complex vascular blueprints, and a mathematical means to design a second vascular architecture that does not intersect the first. FIGS. 3A-D illustrate adaptations of mathematical space-filling curves into entangled vessel topologies within hydrogels (20 wt% PEGDA, 6 kDa), which are not according to the claimed invention and thus to be seen as reference-embodiments. These include a helix surrounding an axial vessel (FIG. 3A), 1 and 2 interpenetrating Hilbert curves (FIG. 3B), a bicontinuous cubic lattice (based on a Schwarz P surface, FIG. 3C), and a torus entangled with a torus knot (FIG. 3D). Perfusion with colored dyes and micro-computed tomography (µCT) analysis demonstrate pattern fidelity, vascular patency, and fluidic independence between the two networks.

The efficiency of the intervascular interstitial transport was evaluated by measuring the delivery of oxygen from a source vessel to perfused human red blood cells (RBCs) flowing in an adjacent 3D topology. FIGS. 3E-F illustrate the tessellation of the axial vessel and its encompassing helix along a serpentine pathway while maintaining the intervessel distance at 300 µm. The top-down photograph of FIG. 3E (with magnified view shown in FIG. 3F) is of a fabricated hydrogel with oxygen and RBC delivery to respective vessels. Perfusion of deoxygenated RBCs (pO₂ ≤ 40 mmHg; sO₂ ≤ 45%) into the helical channel during ventilation of the serpentine channel with humidified gaseous oxygen (7 kPa) caused a noticeable color change of RBCs from dark red at the inlet to bright red at the outlet.

Collection of perfused RBCs showed significantly higher oxygen saturation and oxygen partial pressure compared to deoxygenated RBCs loaded at the inlet, and compared to negative control gels ventilated with humidified nitrogen gas. This is illustrated by FIG. 3G, for which perfused RBCs were collected at the outlet and quantified for sO₂ and pO₂. Compared to deoxygenated RBCs perfused at the inlet (dashed line), oxygen flow increased sO₂ and pO₂ of perfused RBCs compared to nitrogen flow negative control.

Oxygenation of perfused human red blood cells in serpentine-helix gels are affected by flow rates. The experimental results of FIGS. 3H-I show that slower perfusion of deoxygenated RBCs through the vascular network (during gaseous oxygen flow) resulted in higher sO₂ (B) and pO₂ of the collected RBCs. The dashed line indicates the measured sO₂ and pO₂, respectively, of deoxygenated RBCs used for the perfusion. FIG. 3J shows that the fraction of oxygenated hemoglobin increased significantly with slower flow rates. FHHb = fractional deoxyhemoglobin; FMetHb = fractional methemoglobin; FCOHb = fractional carboxyhemoglobin; FO₂Hb = fractional oxyhemoglobin. These data illustrate the viability of the hydrogel devices for use as channels for carrying RBCs.

The following establishes the utility of the described processes for fabricating structurally complex and functional replacement tissues for therapeutic transplantation. The liver is the largest solid organ in the human body, carrying out hundreds of essential tasks in a manner that is thought to be intimately dependent upon its structural topology, and is the organ used for these studies.

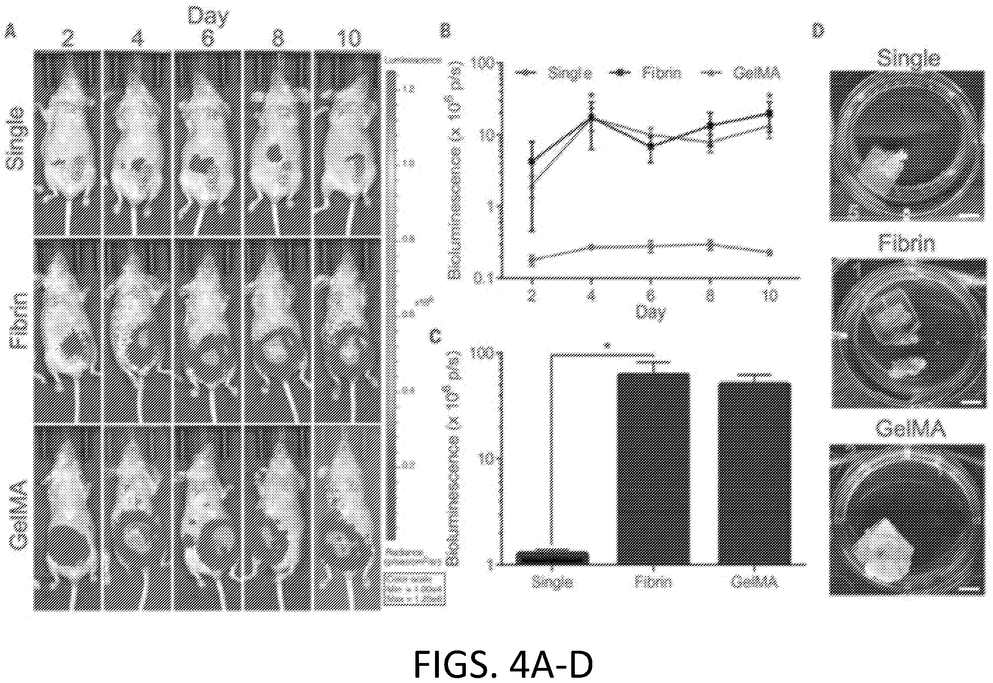

Complex structural features in hydrogels were created within the expanded design space imparted by SLATE (described below) to assemble multi-material liver tissues. Also this embodiment is to be considered as a reference-embodiment, not according to the claimed invention. Bioprinted single-cell tissues and bioprinted hydrogel 'carriers' containing hepatocyte aggregates were fabricated (FIG. 4A-C). Albumin promoter activity was enhanced in hydrogel carriers containing hepatic aggregates after implantation in nude mice. Data from all time points for each condition are shown in FIG. 4B with cumulative bioluminescence for each condition shown in FIG. 4C. The albumin promoter activity of tissue carriers loaded with aggregates was enhanced by more than sixty-fold compared to implanted tissues containing single cells.

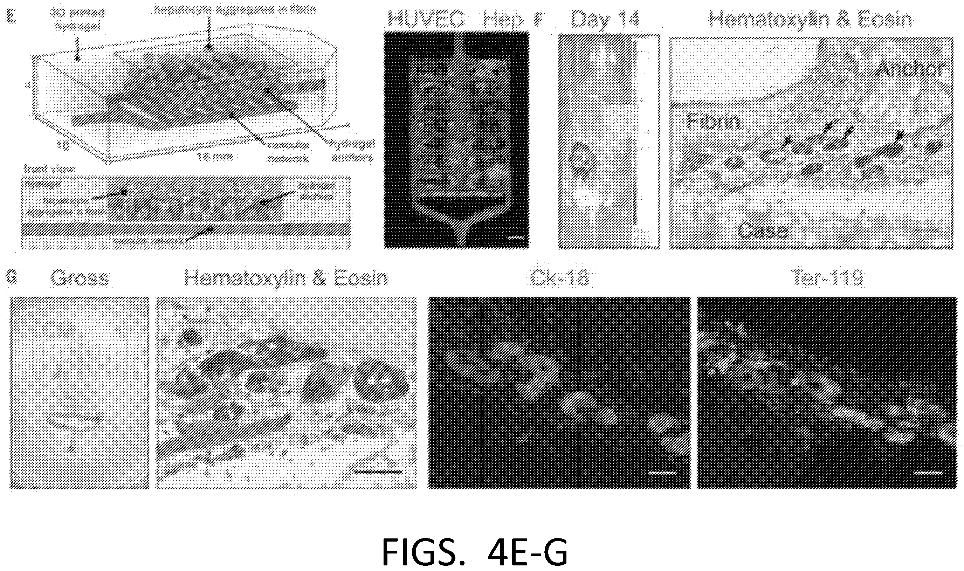

FIG. 4D shows gross examination of tissues after resection, where hydrogel carrier tissues appeared to have more integration with host tissue and blood. Despite the improved utility of hepatic aggregates over single cells, aggregate size puts significant architectural limitations on 3D printing because aggregates are larger in size than typical lowest voxel resolution (50 µm). To accommodate these design constraints, a more advanced carrier which could deliver hepatic aggregates within natural fibrin gel was used, having a vascular compartment that can be seeded with endothelial cells, and incorporates structural hydrogel anchors to physically, rather than chemically, retain the fibrin gel and facilitate remodeling between the graft and host tissue. Referring to FIG. 4E, microchannel networks were seeded with human umbilical vein endothelial cells (HUVECs) while hydrogel anchors physically entrap fibrin gel containing the hepatocyte aggregates (Hep) observed via confocal microscopy.

The bioengineered liver tissues survive transplantation in a model of chronic liver injury. After 14 days of engraftment in mice with chronic liver injury, hepatic hydrogel carriers exhibited albumin promoter activity indicative of surviving functional hepatocytes (FIG. 4F). Immunohistological characterization revealed the presence of hepatic aggregates adhered to printed hydrogel components that stained positively for the hepatocyte marker cytokeratin-18 (FIG. 4F-G). Further characterization through gross examination and higher magnification images of hematoxylin and eosin stained slides indicated the presence of host blood in explanted tissues. Immunostaining for Ter-119 confirmed erythroid identity of cells in microvessels immediately juxtapositional to hepatic microaggregates in explanted tissues (FIG. 4G, right). These illustrate the efficacy of the hydrogel devices for use as implantable devices.

Additional structural features of native distal lung are included in the monolithic bioinspired hydrogel models of alveolar morphology and oxygen transport. 3D hydrogels that contain branching networks and that can support mechanical distension during cyclic ventilation of a pooled airway could enable investigations of the performance of lung morphologies derived from native structure and could provide a broad workflow to develop and interrogate new functional topologies.

Complex morphology are approximated mathematically as 3D space-filling tessellations of polyhedra, which is a morphology used in devices according to the claimed invention. To manufacture a hydrogel matrix construct that models alveolar morphology and oxygen transport a 3D model of the hydrogel construct is created using a design software. The construct includes a first computational algorithm that yields a first tubular channel network and a second computational algorithm, different from the first computational algorithm, that yields a second tubular channel network. The first and second tubular channel networks are two independent, entangled vascular networks (e.g., alveolar morphology and oxygen transport morphology). The 3D model is converted to a format suitable for use in a 3D additive manufacturing software to yield a formatted 3D device to be generated using a 3D additive manufacturing machine.

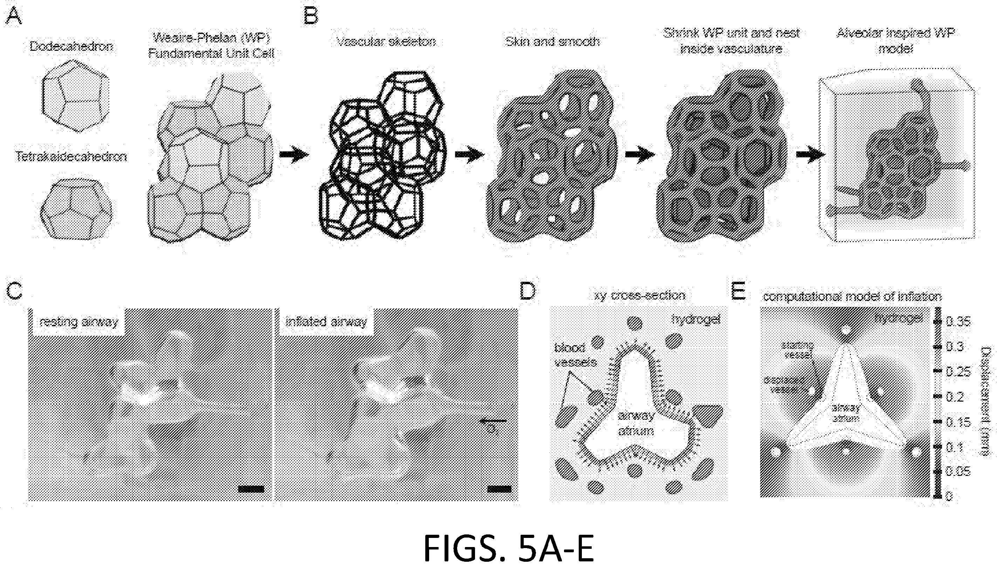

Steps of creating a tessellated model include fusing multiple spheres in a radially symmetric fashion to create an airway topology, offsetting the airway topology to generate a vascular surface topology, (by moving each face in its local normal direction and having the new surface serve as the template on which a new vascular skeleton is built), tessellating, skelatonizing, and skinning the vascular surface topology to produce a Voronoi vascular topology. Tessellating includes performing a tessellation of polyhedra, and a Boolean subtraction of the original airway and the tessellated Voronoi vascular topology from a solid volume to generate a final 3D model for additive manufacturing. FIGS. 5A-G illustrate these details for the design of bioinspired alveoli models. In FIG. 5A the fundamental Weaire-Phelan polyhedra are tessellated in 3D to fill space. FIG. 5B illustrates the procedural derivation of the entangled alveolar model from the Weaire-Phelan tessellation. The manifold air surface is extended in the normal direction, faces removed, and edges ensheathed in a smoothed polygonal mesh to form a highly branched vascular network (containing 185 vessel segments and 113 fluidic branch points) that encloses the airway and tracks its curvature. FIG. 5C are photographs of the printed alveoli model, without RBC flow in the ensheathing vasculature, highlighting that the airway is smaller at rest (left) compared to the airway during inflation and hydrogel distension (right, 10 kPa, 0.5 Hz). Cyclic ventilation of the pooled airway with humidified oxygen gas (10 kPa, 0.5 Hz) results in noticeable distension and an apparent change in the curvature of concave airway regions.

FIG. 5D is a schematic xy cross-section view of the model with convex (blue) and concave (green) regions of the airway (white), and accompanying blood vessels (red). Normal force arrows converge at concave regions. This simplified 2D computational model of airway inflation predicts anisotropic distension of the airway and compression of adjacent blood vessels corresponding to local curvature. This is shown in FIG. 5E, a 2D computational model of a symmetric airway surrounded by circular vessels equidistant from convex or concave regions of the airway (black edges indicate starting positions). Upon airway inflation, concave regions displace and compress adjacent blood vessels.

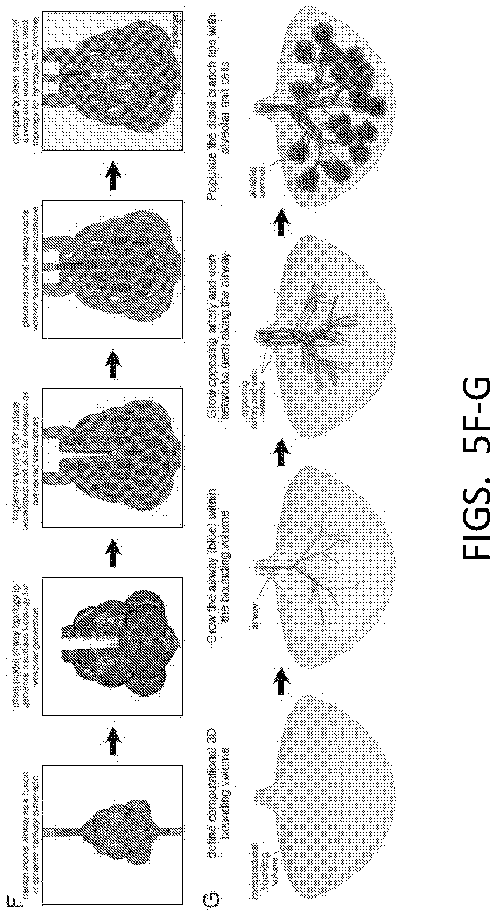

In FIG. 5F the steps of distal lung subunit model design are illustrated. The airway was first designed by fusion of spheres with inlet and outlet channels. The airway topology was offset and used as a template for vascular generation by implementing a Voronoi 3D surface tessellation, skeletonization, and skinning. A Boolean subtraction of these two topologies from a solid volume was performed which represents the final hydrogel design.

FIG. 5G illustrates generation of the lung- mimetic model by populating branch tips of airway and offset vascular networks, grown within a defined computational 3D bounding volume, with the distal lung subunit model.

Alveolar morphology are approximated mathematically as 3D space-filling tessellations of polyhedra that are efficiently space-filling and also replicate an ensheathing vasculature that closely tracks the curvature of the 3D airway topography. FIGS. 6A-I illustrate a bioinspired alveolar model with an ensheathing vasculature from 3D tessellations of the Weaire-Phelan topology. FIG. 6A shows the architectural design of an alveolar model topology based on a Weaire-Phelan 3D tessellation and a topologic offset to derive an ensheathing vasculature. The cutaway view at the bottom illustrates the model alveoli with a shared airway atrium. The 3D tessellations produce a surface containing both convex and concave regions that are reminiscent of native alveolar air sacs with a shared airway atrium supporting alveolar buds.

FIG. 6B shows printed hydrogels (20 wt% 6 kDa PEGDA) patterned with the alveolar model topology of FIG. 6A at a voxel resolution of 5 pL and a print time of 1 hr. The figure is of the printed hydrogel during RBC perfusion while the air sac is ventilated with O₂. Perfusion of deoxygenated RBCs at the blood vessel inlet (10-100 µL/min) during cyclic ventilation led to observable compression and RBC clearance from vessels next to concave airway regions (FIGS. 6B, C). With dilute RBC streams at the early stages of perfusion the cyclic compression of RBC vessels-actuated by the concave airway regions upon each inflation cycle-acted as switching valves to redirect fluid streams to neighboring vessel segments.

Analysis from a 3D computational model supports anisotropic distension of the concave regions of the airway during inflation (FIG. 6D). Moreover, despite the alveolar model hydrogel (0.8 mL) being more than four times smaller in volume than the serpentine-helix model (3.5 mL), the result is similar oxygenation efficiencies between the two designs (FIG. 6E). These data suggest that branching topology, hydrogel distension, and the redirection of fluid streams during ventilation may boost intravascular mixing and allow faster volumetric uptake of oxygen by the well-mixed RBCs. These data illustrate the advantage of vascular constriction during breathing actualized in biocompatible materials and within aqueous environments.

For a scalable lung-mimetic design, the location of the vascular inlet, the vascular outlet, and the air duct are consolidated such that distal lung subunits can be populated on the tips of multiscale branching architecture. FIG. 6F shows a branching airway within a chosen computational bounding volume. This is an elaboration of a lung-mimetic design through generative growth of the airway, offset growth of opposing inlet and outlet vascular networks, and population of branch tips with a distal lung subunit.

The centerlines of inlet and outlet blood vessel networks are grown opposing each other across and topologically offset from the airway, and the blood vessels traverse down to the tips of all daughter branches. The tips of each distal lung are populated with an alveolar unit cell (FIG. 6G) whose ensheathing vasculature (containing 354 vessel segments and 233 fluidic branch points) itself is an anisotropic Voronoi surface tessellation along a topological offset of its local airway. Hydrogels (20 wt% 6 kDa PEGDA) advantageously were found to withstand more than 10,000 ventilation cycles (at 24 kPa and a frequency of 0.5 Hz) over 6 hours during RBC perfusion and while switching the inflow gas between humidified oxygen and humidified nitrogen (FIG. 6H-J). FIG. 6I shows color-filtered views of the early stages of RBC perfusion and demonstrates that ventilation promotes RBC mixing and bidirectional flows within selected vessel segments near the midpoint of the distal lung subunit.

Referring back to FIG. 6E, FIGS. 7A-E show additional imaging and development of hepatic hydrogel carriers, not according to the claimed invention. In FIG. 7A, 3D printed hydrogel carriers accommodate primary hepatocyte aggregates (green) suspended in fibrin (blue), entangled with hydrogel anchors (purple) and has an underlying patterned vascular network (red). FIG. 7B shows 3D printed chamber demonstrate perfusable vasculature (red), and hydrogel anchors. FIG. 7C is a magnified view of highlighted region from FIG. 7B showing hydrogel anchors and underlying perfusable vasculature (red). FIG. 7D is a 250 µm cross-section view demonstrates the fibrin seeding volume (blue) and underlying vasculature (red). FIG. 7E is a confocal maximum intensity projection (Max IP, left) and volumetric rendering of endothelial cords (red) and hepatocytes (green).

SLATE studies indicate that SLATE fabrication supports rapid biomanufacturing, can maintain the viability of mammalian cell lines, supports the normal function and differentiation of primary human stem cells, and suggests an experimentally tractable means to explore stem cell differentiation as a function of soluble factor delivery via vascular perfusion.

FIG. 8A is a schematized workflow for fabrication of 3D objects by projection stereolithography, in accordance with various embodiments. Photomasks corresponding to a 3D model can be projected into a vat containing a photosensitive liquid solution. Upon completion of sequential layer-by-layer photopatterning, a 3D object is obtained. FIG. 8B is a 3D rendering of an example projection stereolithography apparatus 800. The apparatus can include a motor 810 (which can be attached to a frame 840), controlled by an actuator 815, onto which the build platform 820 is attached. The back of the printer can house the motherboard 825 that controls the motor and performs input/output with any additional sensors or switches. The front of the printer contains a mirror 830 placed to reflect incident patterns from a projector 835 onto a dish containing the photosensitive materials. All off-white colored parts correspond to parts that were 3D printed out of consumer-grade poly(lactic acid) (PLA) plastic filament. FIG. 8C Schematic demonstrating photomasks (left) that are generated at different locations along the height of the 3D rook model.

The device fits entirely inside common biosafety cabinets with sufficient room for cell handling. Although SLATE fabrication is rapid (at up to 12 mL/hr with voxels of 250 pL), mammalian cells typically settle out of suspension within tens of seconds. To prevent cells from settling and facilitate automated bioprinting, xanthan gum (a natural, biocompatible, shear-thinning carbohydrate) is added to the pre-hydrogel solution.

SLATE demonstrates the ability to generate tissue constructs containing mammalian cells using the custom lung-mimetic architectures described above. Hydrogels composed of tens of layers can be printed in a few minutes, and mammalian cells are uniformly distributed (FIGS. 9A, B). FIGS. 9A, B show that incorporation of xanthan gum into the pre-hydrogel solution resulted in homogeneous distribution of cells along the height of the gel. FIG. 9B is a side view (xz plane) of a fabricated 20 wt% hydrogel containing HEK mCherry cells (20×10⁶ cells/mL). The experimental results in FIGS. 9C, D show total light output of HEK Luc2P cells encapsulated in PEGDA hydrogels containing a horizontal 0.8 mm diameter channel at different cell densities (FIG. 9C) and different flow rates of luciferin substrate (FIG. 9D).

FIGS. 10A-C illustrate rapid biomanufacturing with cryopreserved cell stocks and non-invasive characterization of cellular activity. FIG. 10A illustrates that cryopreserved cell stocks are thawed and immediately used in projection stereolithography to yield perfusable tissues with patterned vessel architectures. The total procedure from cell thaw to perfusion experiments takes less than 1 hr. Referring to FIG. 10B, using this workflow, fabricated tissue constructs containing mammalian cells (2.5×10⁷ cells/mL) expressing firefly luciferase (Luc2P) were perfused with deoxygenated or oxygenated RBCs along with luciferin substrate. Luminescence quantification (3 hr) demonstrates increased light emission from cells within gels perfused with oxygenated RBCs. FIG. 10C is a full 3 hour time course of the perfusion experiment from FIG. 10B plotted as total light flux emitted from cells within hydrogels.

Hydrogel constructs can also be populated with human lung fibroblasts in the bulk of the interstitial space and human epithelial-like cells in the airway which could facilitate the development of a hydrogel analog of a lab-on-a-chip lung design for a monolithic lung-mimetic perfusion and ventilation tissue culture system. FIG. 11A illustrates a bioinspired design of an alveolar sac with surrounding pulmonary vasculature. The overall size of the model is 9×11.8×4.3 mm (x,y,z) with a smallest channel diameter of 400 µm and alveolus diameter of 600 µm. FIG. 11B is a photo of the fluidic connections to the printed gel for perfusion through the vascular channel and ventilation of the airway vessel. FIG. 11C shows hydrogels that were fabricated with IMR-90 fibroblasts (10×10⁶ cells/mL) encapsulated in the bulk of the hydrogel, including the interstitial region. Ventilation of the airway under perfusion tissue culture through the vasculature (20 µL/min over 5 days) maintained patency of the airway as seen in cross-sectional imaging after staining for nuclei (Hoechst). In FIG. 11D, seeded A549 epithelial-like cells (expressing H2B-mVenus, center) are attached to the airway lumen of printed hydrogels containing encapsulated fibroblasts in the interstitial zone.

FIGS. 12A, B show histological results of a related multi-week tissue culture of hMSCs with osteogenic differentiation media, in which osteogenic marker-positive hMSCs were visible throughout the perfused single channel gels. FIG. 12A shows alkaline phosphatase (ALP) staining of hMSC tissue cross-section from a hydrogel that underwent osteogenic media perfusion for 14 days, with the vascular channel highlighted with the dotted circle (FIG. 12B being a close-up of the section). ALP stain was found strongly along the perimeter of the perfused channel with some positive staining throughout the bulk of the gel.

As mentioned above, synthetic and natural food dyes can be used as biocompatible photoabsorbers to enable the stereolithographic production of the hydrogels containing intricate and functional vascular architectures. Aqueous pre-hydrogel solutions containing tartrazine (yellow food coloring FD&C Yellow 5, E102), curcumin (from turmeric), or anthocyanin (from blueberries) can each yield hydrogels with a patent vessel. In addition to these organic molecules, inorganic gold nanoparticles (50 nm), widely regarded for their biocompatibility and light attenuating properties, also function as an effective photoabsorbing additive to generate perfusable hydrogels (discussed below with respect to FIG. 15).

Possible photoabsorbers can be one or more food dyes including tartrazine, Sunset Yellow FCF (Yellow No. 6), Brilliant Blue FCF (FD&C Blue No. 1), Indigo Carmine (FD&C Blue No. 2), Fast Green FCF (FD&C Green No. 3) anthocyanins, anthocyanidin, erythrosine (FD&C Red No.3), Allura Red AC (FD&C Red No. 40), riboflavin (Vitamin B2, E101, E101a, E106), ascorbic acid (vitamin C), Quinoline Yellow WS, carmoisine (azorubine), Ponceau 4R (E124), Patent Blue V (E131), Green S (E142), Yellow 2G (E107), Orange GGN (E111), Red 2G (E128), caramel color, phenol red, methyl orange, 4-nitrophenol, and NADH disodium salt. Also possible are curcumin (E100), turmeric, alpha carotene, beta carotene, canthaxanthin (keto-carotenoid), cochineal extract, paprika, saffron, ergocalciferol (vitamin D2), cholecalciferol (vitamin D3), Citrus Red 2, annatto extract, and lycopene.

FIGS. 13A-E illustrate the fabrication of patent vessels within monolithic hydrogels. FIG. 13A schematically illustrates a monolithic hydrogel with a horizontal vessel that can be fabricated by projection stereolithography with one of the suitable photoabsorbers (tartrazine is used for the experimental results shown) added to the pre-hydrogel solution to minimize excess light penetration into the nascent vessel. FIG. 13B shows that projection stereolithography with tartrazine yields hydrogels with minimal excess crosslinking in the vessel lumen. FIG. 13C is absorbance spectra of a LAP photoinitiator and the photoabsorber tartrazine. The absorbance spectra of tartrazine encompass the light source used (vertical bar at 405 nm), which can be used to initiate photocrosslinking via the LAP photoinitiator. FIG. 13D is photorheology during short duration (left) and long duration (right) light exposures (blue shaded region) and demonstrates that tartrazine addition (0-3 mM) slows the induction of crosslinking but does not ultimately interfere with gelation. FIG. 13E shows that hydrogels release up to 70% tartrazine within hours (exemplified by photos of gels above a color wheel), making fabricated hydrogels suitable for color and fluorescence imaging.

To understand how these photoabsorbers impact the gelation kinetics of photopolymerizable hydrogels photorheological characterization was performed with short duration light exposures. The results indicate that these additives cause a dose-dependent delay in the induction of photocrosslinking (FIGS. 13D and 15E). Saturating light exposures that extend beyond the reaction termination demonstrate that suitable additives did not ultimately interfere with the reaction because hydrogels eventually reached an equivalent storage modulus independent of the additive concentration (FIGS. 13D and 15E).

FIGS. 14A-D show experimental results indicating that fabrication orientation and layer thickness affect the fidelity of printed channels. FIG. 14A is a circular channel of 1 mm diameter fabricated such that the longitudinal axis of the channel is parallel with the print direction. This orientation produces no overhanging features with high circularity (as quantified in FIG. 14D). FIG. 14B is a square channel with 1 mm sides, fabricated in a horizontal orientation perpendicular to the light projection axis (the asterisk denotes the region where excess light penetration caused extraneous hydrogel crosslinking). FIG. 14C shows circular channels of 1 mm diameter in a horizontal orientation that were printed using a layer thickness of 200, 100, 50, or 25 µm. FIG. 14D is a plot of channel circularity as a function of layer thickness. Horizontal channel prints are shown left of the dashed line and the vertical channel from FIG. 14A is shown at right. As layer height decreases, channels exhibit higher circularity and thus greater geometric fidelity to their original CAD model.

FIGS. 15A-G show experimental results indicating that biocompatible photoabsorbers allow fabrication of hydrogels with open channels. FIG. 15A are absorbance spectra of LAP photoinitiator and biocompatible photoabsorbers tartrazine, curcumin, anthocyanin, and 50 nm gold nanoparticles. The absorbance spectra of these additives encompass the light source (vertical bar at 405 nm) that can be used to initiate photocrosslinking via the LAP photoinitiator. FIG. 15B shows that the amount of tartrazine was found to be lower in a printed gel (4×4×2 mm with 1 mm diameter channel) compared to the starting pre-hydrogel solution, attributable to its degradation during the radical-mediated photochemical reaction. FIG. 15C is a hydrogel with the horizontal vessel geometry described in (FIG. 1A) but fabricated without any photoabsorber additive, results in undesired gelation within the vessel, completely occluding it. Rhodamine-dextran was incorporated into the pre-hydrogel mixture to enable fluorescence. FIG. 15D is fluorescence (left) and color photograph (right) images of curcumin (top), anthocyanin (middle), and 50 nm gold nanoparticles (bottom) as photoabsorbers for fabrication of open horizontal channels using stereolithography. FITC-dextran was incorporated into the pre-hydrogel mixture to obtain fluorescence images. FIG. 15E is the results of photorheological studies with short (left) and long (right) duration light exposures with the following additives: curcumin (top), anthocyanin (middle), and 50 nm gold nanoparticles (bottom). FIG. 15F are working curves for hydrogel formulations of increasing photoabsorber concentration. Each data point represents the time required for a single layer hydrogel of given thickness to reach its gel point during dynamic oscillation and irradiation and all data points are shown in table form in FIG. 15G.

Tartrazine is selected as an example of a preferred photoabsorber. In addition to its low toxicity in humans and broad utility in the food industry, the hydrophilic dye is easily washed out of generated hydrogels (for example, 70% elutes within 3 hours for small gels) resulting in nearly transparent constructs which are suitable for imaging. Some tartrazine may also be degraded during polymerization as tartrazine is known to be sensitive to free radicals. Submerging gels in water or saline solution to remove soluble tartrazine also flushes the vascular topology and removes unreacted pre-hydrogel solution. In contrast to tartrazine, curcumin is lipophilic and does not wash out in aqueous solutions, anthocyanin has a peak absorbance far away from our intended 405 nm light source requiring high concentrations for suitable potency, and gold nanoparticles are physically entrapped and make transmission or fluorescence microscopy impractical. Use of tartrazine is advantageous in the hydrogel pre-polymerization solution as a light-absorbing additive material suitable to control light penetration that can be later removed from the device formed of the pre-polymerization solution, allowing it become transparent or nearly so (e.g., allowed 90% or more of incident light to pass through).

The hydrogel devices discussed herein can further include cells (plant, bacterial, or mammalian) either in the pre-polymerization solution or later seeded in the porous hydrogel device. FIGS. 16A-D show experimental results indicating that the inclusion of tartrazine does not affect viability and differentiation of human mesenchymal stem cells. In FIGS. 16A, B human mesenchymal stem cells (hMSCs) incubated with 1-10 mM tartrazine (FIG. 16 A) and 10-50 mM LAP (FIG. 16B) for 20 and 60 min resulted in high cell viability. FIG. 16C shows that hMSCs within bioactive cylindrical hydrogels demonstrated high cell viability. FIG. 16D is quantification of osteogenic differentiation of hMSCs encapsulated in hydrogels fabricated with tartrazine and show that the cells within cylindrical fabricated hydrogels remain viable and can undergo osteogenic differentiation.

More advanced photoactive materials can be used to create the hydrogel devices. FIGS. 17A-E show experimental results illustrating the compatibility of photoabsorber additives with advanced materials and fabrication strategies. FIG. 17A shows photorheology of radical-mediated chain-growth photopolymerization with PEGDA and photoabsorber and reveals that increases in hydrogel stiffness slowly increase long after light (blue shaded region) is switched off, also known as dark reactions. In contrast, as shown in FIG. 17B, with PEG-norbornene and PEG- dithiol step-growth polymerization (thiol-ene click chemistry) in the presence of photoabsorber additives, an increase in hydrogel stiffness stops abruptly after light (shaded blue region) is switched off. FIG. 17C shows that the staircase-like appearance of step-growth reaction kinetics can be replicated in series with discrete light pulses (shaded blue regions) to yield hydrogels of variable stiffness without the complication of slowly evolving reactions as seen in chain-growth polymerization. FIG. 17D shows that projection stereolithography of thiol-ene materials yields hydrogels with minimal excess crosslinking in the vessel lumen. FIG. 17E illustrates that continuous projection stereolithography of hydrogels containing open channels can be achieved. FITC-Dextran was incorporated into the pre- hydrogel mixture to obtain the fluorescence image.

Strong lamination was observed between adjacent fabricated layers and a rapid response of the patterned hydrogel to mechanical deformations (as shown in the resting/inflated results of FIG. 2B). This facile generation of soft hydrogels with patent cylindrical vessels oriented orthogonal to the light projection axis suggests a significant design flexibility toward the generation of complex vascular topologies, and the optical clarity of resultant hydrogels implies imaging methodologies suitable for characterization and validation of fluid flows.

The methods and validation experiments describe how to fabricate a 3D hydrogel construct by creating a 3D model of the construct based on a tessellation of polyhedra having a number of faces connected by edges and vertices. Then by generating a vascular component of the model by removing the faces and optionally the vertices of the polyhedra, and using the remaining topology as a vascular skeleton, skinning the skeleton with a polygonal mesh, and then smoothing the result to produce cylindrical channels along the edges of the model with intervessel junctions located at each vertex location. An airway component of the model can be generated by scaling the faces of the 3D model along local face normals such that the airway is nested inside the vasculature (vascular) component, forming independent fluidic connections to the vasculature and to the airway, and performing a Boolean subtraction from a solid volume, and combining the vascular component and the airway component of the model.

These 3D models can be used for additive manufacturing with a pre-polymerization solution including a photoabsorber solution being supplied to the 3D additive manufacturing machine. The pre-polymerization solution can have one or more bacterial, mammalian, and plant cells or the architecture resulting from the 3D model can be a first tubular channel network or second tubular channel network that is lined with cells. Alternatively, the hydrogel matrix can be embedded with cells. The cells can be one or more of liver, lung, bone, endothelial, cardiac, pancreas, kidney, epithelial, muscle, cartilage, stem cells, skin, or eye cells.

While the 3D additive manufacturing machine generates the hydrogel based on the 3D model, heat can be added via a silicone heater, heat lamps, or infrared LEDs. The building hydrogel can be enclosed in a heating enclosure during generation. Once fabricated, the hydrogel device can be implanted into a subject for medical intervention, or multiple devices joined into a larger implant can be used for treatment.

In accordance with various embodiments, a device including a hydrogel matrix as defined in the appended claims is provided. The hydrogel matrix includes a photoabsorber and a void architecture in the matrix, having a first vessel architecture and a second vessel architecture that are each tubular and branching, wherein the first and second vessel architectures are fluidically independent from each other, and wherein one or both of the first vessel architecture and second vessel architecture is formed from a model based on a tessellation of polyhedra.

In accordance with various embodiments, the device allows between about 50% and 99.9% of visible light (400 - 700 nm wavelength) incident on the device to pass therethrough and allow imaging of the device. In accordance with various embodiments, the device allows between about 60% and 99.8%, between about 70% and 99.6%, between about 80% and 99.5%, or between about 85% and 99.0%, of visible light incident on the device to pass therethrough and allow imaging of the device. In accordance with various embodiments, the device allows at least 50%, 55%, 60%, 65%, 70%, 75%, 80%, 85%, 90%, or 90%, inclusive of any percentages of visible light incident on the device to pass therethrough and allow imaging of the device. In accordance with various embodiments, the photoabsorbers might not result in transparent hydrogels.

In accordance with various embodiments, the photoabsorber has been at least partially washed out of the device. For example, some percentage of the photoabsorber may not wash out completely. This can result, for example, in gels that are transparent but still slightly "yellow" in color. In accordance with various embodiments, the photoabsorber has been washed out at least 70%, at least 75%, at least 80%, at least 85%, at least 90%, at least 95%, or at least 99%, inclusive of any values therebetween. In accordance with various embodiments, the photoabsorber is degradable independent of any degradation of the remaining hydrogel matrix. In accordance with various embodiments, the photoabsorber is degradable by chemical or physical processes. For example, at least 70% of a photoabsorber such as tartrazine can be washed within three hours after printing is complete.

In accordance with various embodiments, the photoabsorber is photobleachable by exposure to absorbable light having a wavelength 365 - 450 nm wavelength, chemical degradation such as by peroxides, or any other suitable material, or removable by exposure to boiling aqueous solution, such as water, or any other suitable material.

According to the claimed invention, one or both of the first vessel architecture and second vessel architecture is formed from a model based on a tessellation of polyhedra. In accordance with various embodiments, the model based on a tessellation of polyhedra represents blood vessels. In accordance with various embodiments, the model based on a tessellation of polyhedra represents an airway.

In accordance with various embodiments, the void architecture is multi-vascular and further comprises a functional valve. In accordance with various embodiments, the valve is a bicuspid valve, tricuspid valve, monocuspid valve, or Tesla valve.

In accordance with various embodiments, the void architecture comprises a fluidic static mixer. In accordance with various embodiments, the fluidic static mixer has between two and fifty (2-50) fin elements. In accordance with various embodiments, the fluidic static mixer has between 2-50, 3-40, 4-30, 5-25, 6-20, 7-15, or 8-12 fin elements, inclusive of any ranges therebetween. In accordance with various embodiments, the functional valve is positionable at any position in the multi-vascular void architecture.

In accordance with various embodiments, the device is monolithic. In accordance with various embodiments, the hydrogel matrix is a photopolymerized hydrogel matrix and/or biodegradable. In accordance with various embodiments, the device is produced by additive manufacturing.

In accordance with various embodiments, the first vessel architecture and the second vessel architecture are entangled. In accordance with various embodiments, the first vessel architecture is ensheathed by the second vessel architecture. In accordance with various embodiments, the hydrogel matrix is porous. In accordance with various embodiments, the porosity ranges from about 10 nm (100 Angstroms) to about 10 µm, 20 nm to about 8 µm, 30 nm to about 7 µm, 40 nm to about 6 µm, 50 nm to about 5 µm, 60 nm to about 4 µm, 70 nm to about 3 µm, 80 nm to about 2 µm, or 100 nm to 1 µm, inclusive of any porosity ranges therebetween. In accordance with various embodiments, the device is implantable. In accordance with various embodiments, the photoabsorber is hydrophilic.

In accordance with various embodiments, the photoabsorber is one of a food dye, tartrazine, sunset yellow FCF (Yellow No. 6), Brilliant Blue FCF (FD&C Blue No. 1), Indigo Carmine (FD&C Blue No. 2), Fast Green FCF (FD&C Green No. 3) anthocyanins, anthocyanidin, erythrosine (FD&C Red No.3), Allura Red AC (FD&C Red No. 40), riboflavin (Vitamin B2, E101, E101a, E106), ascorbic acid (Vitamin C), Quinoline Yellow WS, carmoisine (azorubine), Ponceau 4R (E124), Patent Blue V (E131), Green S (E142), Yellow 2G (E107), Orange GGN (E111), Red 2G (E128), caramel color, phenol red, Methyl orange, 4-nitrophenol, and NADH disodium salt. In accordance with various embodiments, the photoabsorber can include a concentration of about 0.1 mM to about 100 mM, about 0.2 mM to about 90 mM, about 0.3 mM to about 80 mM, about 0.4 mM to about 70 mM, about 0.5 mM to about 60 mM, about 0.6 mM to about 50 mM, about 0.7 mM to about 40 mM, about 0.8 mM to about 30 mM, about 0.9 mM to about 20 mM, about 1.0 mM to about 10 mM, inclusive of any concentration ranges therebetween.

In accordance with various embodiments, the photoabsorber is hydrophobic. In accordance with various embodiments, the photoabsorber is one of Curcumin (E100), turmeric, alpha carotene, beta carotene, canthaxanthin (keto-carotenoid), cochineal extract, paprika, saffron, ergocalciferol (vitamin D2), cholecalciferol (vitamin D3), Citrus Red 2, annatto extract, and Lycopene. In accordance with various embodiments, the hydrophobic photoabsorber can include a concentration of about 0.1 mM to about 100 mM, about 0.2 mM to about 90 mM, about 0.3 mM to about 80 mM, about 0.4 mM to about 70 mM, about 0.5 mM to about 60 mM, about 0.6 mM to about 50 mM, about 0.7 mM to about 40 mM, about 0.8 mM to about 30 mM, about 0.9 mM to about 20 mM, about 1.0 mM to about 10 mM, inclusive of any concentration ranges therebetween. In accordance with various embodiments, the hydrophobic photoabsorber can include a solvent. The solvent can be selected from the group consisting of dimethyl sulfoxide, ethanol (50-100%), isopropyl alcohol, (50-100%) dimethylformamide, N-Methyl-2-pyrrolidone, tetrahydrofuran, chloroform, methylene chloride, toluene, hexafluoro-2-propanol, and any combination thereof.

In accordance with various embodiments, the device includes three or more vessel architectures fluidically independent from each other and from the first and second vessel architectures.

In accordance with various embodiments, the fluidic static mixer includes a photoabsorber. In accordance with various embodiments, the fluidic static mixer includes tartrazine. In accordance with various embodiments, the fluidic static mixer includes fin elements with 180° twists. In accordance with various embodiments, the fluidic static mixer includes fin elements having a twist angle in the of 30°-180°, 40°-180°, 45°-180°, 50°-180°, 60°-180°, 65°-180°, 70°-180°, 75°-180°, 80°-180°, 85°-180°, 90°-180°, 120°-180°, or 150°-180°, inclusive of any ranges of twisting angles therebetween. In accordance with various embodiments, the fluidic static mixer is produced by additive manufacturing.

In accordance with various embodiments, the device can include a thickener to prevent cell settling. In accordance with various embodiments, the thickener includes xanthan gum having a concentrations about 0.02 wt% to about 2 wt%, about 0.03 wt% to about 1.5 wt%, about 0.04 wt% to about 1.0 wt%, or about 0.05 wt% to about 0.5 wt%, inclusive of any thickener values therebetween.

In accordance with various embodiments, a pre-polymerization solution is provided. In accordance with various embodiments, the pre-polymerization solution includes a photosensitive polymer, and a photoabsorber additive material suitable to control light penetration, wherein the additive material is at least partially removable from a solid formed of the pre-polymerization solution. In accordance with various embodiments, a photosentive polymer is one that can be polymerized via photosensitive reactions. In accordance with various embodiments, photoabsorbers can include photoabsorbers that are covalently or non-covalently bound to polymers. In accordance with various embodiments, the pre-polymerization solution can also include photoinitiators. In accordance with various embodiments, the photoinitiators can be covalently or non-covalently bound to polymers.

In accordance with various embodiments, the pre-polymerization solution can include a thickener to prevent cell settling. In accordance with various embodiments, the thickener includes xanthan gum having a concentrations about 0.02 wt% to about 2 wt%, about 0.03 wt% to about 1.5 wt%, about 0.04 wt% to about 1.0 wt%, or about 0.05 wt% to about 0.5 wt%, inclusive of any thickener values therebetween.

In accordance with various embodiments, the pre-polymerization solution includes a pH between about 1 to about 10, about 1.5 to about 9.8, about 2.5 to about 9.5, about 3.5 to about 9.3, about 4.5 to about 9, about 5.5 to about 8.8, about 6 to about 8.6, or about 6.5 to about 8.5, inclusive of any pH values therebetween.

In accordance with various embodiments, the photosensitive polymer can include polyethylene glycol diacrylate, gelatin methacrylate, collagen methacrylate, silk methacylate, hyaluronic acid methacrylate, chondroitin sulfate methacrylate, elastin methacrylate, cellulose acrylate, dextran methacrylate, heparin methacrylate, NIPAAm methacrylate, Chitosan methacrylate, polyethyelene glycol norbornene, polyethylene glycol dithiol, thiolated gelatin, thiolated chitosan, thiolated silk, PEG based peptide conjugates, or any combination thereof.

In accordance with various embodiments, the ranges of polymer in the pre-polymerization solution can range between about 5 wt% to about 30 wt%, about 10 wt% to about 25 wt%, or about 15 wt% to about 20 wt%, inclusive of any ranges therebetween. In accordance with various embodiments, the ranges of photoabsorber in the pre-polymerization solution can include a concentration of about 0.1 mM to about 100 mM, about 0.2 mM to about 90 mM, about 0.3 mM to about 80 mM, about 0.4 mM to about 70 mM, about 0.5 mM to about 60 mM, about 0.6 mM to about 50 mM, about 0.7 mM to about 40 mM, about 0.8 mM to about 30 mM, about 0.9 mM to about 20 mM, about 1.0 mM to about 10 mM, inclusive of any concentration ranges therebetween. In accordance with various embodiments, the photoinitiator can include a concentration of about 5 mM to about 50 mM, about 6 mM to about 40 mM, about 7 mM to about 30 mM, about 8 mM to about 20 mM, about 9 mM to about 30 mM, about 10 mM to about 50 mM, inclusive of any concentration ranges therebetween.

In accordance with various embodiments, the pre-polymerization solution includes plant, bacterial, or mammalian cells. In accordance with various embodiments, the ranges of cells include 1x10⁶-200x10⁶ per milliliter for mammalian and plant cells. In accordance with various embodiments, the ranges of cells can include 1x10⁷-1x10⁸ per milliliter for mammalian and plant cells. In accordance with various embodiments, the ranges of cells can include 1x10³-1x10¹¹per millileter for bacteria and other archae including fungi. In accordance with various embodiments, the ranges of cells can include 1x10⁸-1x10¹¹ for bacteria and other archae including fungi.

In accordance with various embodiments, the photoabsorber additive material is biocompatible. In accordance with various embodiments, the photoabsorber additive material is hydrophilic. In accordance with various embodiments, the photoabsorber additive material is one of a food dye, tartrazine, sunset yellow FCF (Yellow No. 6), Brilliant Blue FCF (FD&C Blue No. 1), Indigo Carmine (FD&C Blue No. 2), Fast Green FCF (FD&C Green No. 3) anthocyanins, anthocyanidin, erythrosine (FD&C Red No.3), Allura Red AC (FD&C Red No. 40), riboflavin (Vitamin B2, E101, E101a, E106), ascorbic acid (Vitamin C), Quinoline Yellow WS, carmoisine (azorubine), Ponceau 4R (E124), Patent Blue V (E131), Green S (E142), Yellow 2G (E107), Orange GGN (E111), Red 2G (E128), caramel color, phenol red, Methyl orange, 4-nitrophenol, and NADH disodium salt.

In accordance with various embodiments, the photoabsorber additive material is hydrophobic. In accordance with various embodiments, the photoabsorber additive material is one of Curcumin (E100), turmeric, alpha carotene, beta carotene, canthaxanthin (keto-carotenoid), cochineal extract, paprika, saffron, ergocalciferol (vitamin D2), cholecalciferol (vitamin D3), Citrus Red 2, annatto extract, and Lycopene. In accordance with various embodiments, the hydrophobic photoabsorber additive material can include a solvent. The solvent can be selected from the group consisting of dimethyl sulfoxide, ethanol (50-100%), isopropyl alcohol, (50-100%) dimethylformamide, N-Methyl-2-pyrrolidone, tetrahydrofuran, chloroform, methylene chloride, toluene, hexafluoro-2-propanol, and any combination thereof.

In accordance with various embodiments, a method of fabricating, with a processor, a 3D hydrogel construct is provided. In accordance with various embodiments, the method includes creating a 3D model of the construct based on a tessellation of polyhedra having a number of faces connected by edges and vertices; generating a first vascular component of the model by: removing the faces and/or the vertices of the polyhedra, and using the remaining topology as a vascular skeleton, and skinning the skeleton with a polygonal mesh and then smoothing the result to produce cylindrical channels along the edges of the model with intervessel junctions located at each vertex location; generating a second vascular component of the model by: scaling the faces of the 3D model along local face normals such that the second vascular component is nested inside the first vascular component, forming independent fluidic connections to the first vascular component and to the second vascular component, and performing a Boolean subtraction from a solid volume; and combining the two vascular components of the model.

In accordance with various embodiments, the method also includes fabricating a hydrogel alveolar construct by fusing multiple spheres in a radially symmetric fashion to create an airway topology; offsetting the airway topology to generate a vascular surface topology; tessellating, skeletonizing, and skinning the vascular surface topology to produce a Voronoi vascular topology, wherein tessellating comprises performing a tessellation of polyhedra; and performing a Boolean subtraction of the original airway and the tessellated Voronoi vascular topology from a solid volume to generate a final 3D model for additive manufacturing. In accordance with various embodiments, the method can further comprise delivering heat while a 3D additive manufacturing machine generates the 3D model.

In accordance with various embodiments, the tessellation of polyhedra is a Weaire-Phelan space-filling foam model of two dodecahedron and six tetrakaidecahedron cells.

In accordance with various embodiments, a method of manufacturing a hydrogel matrix construct is provided. In accordance with various embodiments, the method includes creating a 3D model of the hydrogel matrix construct using a design software, wherein the 3D model of the hydrogel matrix construct comprises a first computational algorithm that yields a first tubular channel network in the hydrogel matrix construct, and a second computational algorithm, different from the first computational algorithm, that yields a second tubular channel network in the hydrogel matrix, wherein the first and second tubular channel networks are two independent, entangled vascular networks; converting the 3D model to a format suitable for use in a 3D additive manufacturing software to yield a formatted 3D model to be generated using a 3D additive manufacturing machine; and directing the additive manufacturing machine to generate the model.

In accordance with the claimed invention, the computational algorithm is a tessellation of polyhedra.

In accordance with various embodiments, the method also includes supplying a pre-polymerization solution to the 3D additive manufacturing machine wherein the pre-polymerization solution includes a photoabsorber. In accordance with various embodiments, the pre-polymerization solution comprises one or more types of bacterial, mammalian, and plant cells.

In accordance with various embodiments, the method includes lining the first tubular channel network or second tubular channel network with cells. In accordance with various embodiments, the method includes embedding the hydrogel matrix with cells. In accordance with various embodiments, the cells are one or more of liver, lung, bone, endothelial, cardiac, pancreas, kidney, epithelial, muscle, cartilage, stem cells, skin, or eye cells. In accordance with various embodiments, the ranges of cells can include 1x10⁶-200x10⁶ per milliliter for mammalian cells. In accordance with various embodiments, the ranges of cells can include 1x10⁷-1x10⁸ per milliliter for mammalian cells.

In accordance with various embodiments, the method includes delivering heat while the 3D additive manufacturing machine generates the 3D model. In accordance with various embodiments, heat is added via a silicone heater, heat lamps, or infrared LEDs. In accordance with various embodiments, the ranges of temperature from heating can include about 27 °C to about 100 °C, about 28 °C to about 95 °C, about 29 °C to about 90 °C, about 30 °C to about 85 °C, about 31 °C to about 80 °C, about 32 °C to about 75 °C, about 33 °C to about 70 °C, about 34 °C to about 65 °C, or about 35 °C to about 60 °C, inclusive of any temperature or ranges of temperature therebetween. In accordance with various embodiments, the method includes enclosing the 3D model in a heating enclosure during generation of the 3D model.

In accordance with various embodiments, a method of fabricating a 3D hydrogel construct is provided. In accordance with various embodiments, the method includes using a computer-implemented process to: create a 3D model of the construct based on a tessellation of polyhedra having a number of faces connected by edges and vertices; generate a first vascular component of the model; generate a second vascular component of the model; and combine the first and second vascular components of the model.

In accordance with various embodiments, using the computer-implemented process to generate the first vascular component of the model includes removing the faces of the polyhedra, and using the remaining vertices and edges of the topology as a vascular skeleton.

In accordance with various embodiments, using a computer-implemented process to generate the first vascular component of the model further includes skinning the skeleton with a polygonal mesh and then smoothing the result to produce cylindrical channels along the edges of the model with intervessel junctions located at each vertex location.

In accordance with various embodiments, using a computer-implemented process to generate the second vascular component of the model includes scaling the faces of the 3D model along local face normals such that the second vascular component is nested inside the first vascular component, forming independent fluidic connections to the first vascular component and to the second vascular component, and performing a Boolean subtraction from a solid volume.

In accordance with various embodiments, the method further includes a hydrogel alveolar construct by: fusing multiple spheres in a radially symmetric fashion to create an airway topology; offsetting the airway topology to generate a vascular surface topology; tessellating, skeletonizing, and skinning the vascular surface topology to produce a Voronoi vascular topology, wherein tessellating comprises performing a tessellation of polyhedral.

In accordance with various embodiments, the method includes performing a Boolean subtraction of the original airway and the tessellated Voronoi vascular topology from a solid volume to generate a final 3D model for additive manufacturing. In accordance with various embodiments, the tessellation of polyhedra is a Weaire-Phelan space-filling foam model of two dodecahedron and six tetrakaidecahedron cells.

Also described herein, but not forming part of the claimed invention, is a method of treating a subject in need thereof by implanting any of the devices into a subject.

In accordance with various embodiments, a device comprising multiple joined subunits is provided. In accordance with various embodiments, each subunit includes device as disclosed herein.

In accordance with various embodiments, an apparatus for manufacturing a hydrogel matrix construct is provided. The apparatus can comprise a frame, a stage with z-axis drive motor attached to the frame, an electronics board for controlling movement of the stage with respect to the frame, a container configured for holding a solution, a projector for projecting images, wherein the images are image slices of a 3D model of the hydrogel matrix construct, and a build platform configured for holding a substrate, wherein the hydrogel matrix construct is formed on the substrate. In accordance with various embodiments, an optional 45° mirror can be configured for reflecting the images projected from the projector into the container.

In accordance with various embodiments, the substrate can include a composition having organic or inorganic glass, with or without a textured surface that is either inert or functionalized with tethered groups. In accordance with various embodiments, the tethered groups can include covalently or non-covalently assist in the attachment of the hydrogel to the substrate, such as 3-(trimethoxysilyl)propyl methacrylate. In accordance with various embodiments, the substrate composition may also contain mica, polycarbonate, polysulfone, polymethyl methacrylate, polystyrene, cyclic olefin copolymer, polyethylene, polypropylene, or quartz with or without a textured surface that can optionally be modified with tethered groups that can covalently or non-covalently assist in the attachment of the hydrogel to the substrate. These various components, in accordance with various embodiments, can all be provided as part of single apparatus (as described here and exemplified in FIG. 18 discussed in detail below). These various components, however, can also be provided in various other configurations whereby any one or more components can be provided as part of further sub-apparatus that interact with the main apparatus to together provide fundamentally the same functionality as those embodiments where all components are provided within the single apparatus. Further, various embodiments can include only a portion of the components provided above as it should not be assumed that each and every component recited is necessary for the proper functionality of an apparatus for manufacturing a hydrogel matrix construct.

In accordance with various embodiments, the solution can be a pre-polymerization solution comprising a photosensitive polymer and a photoabsorber additive material suitable to control light penetration. The additive material can be removable from a solid formed of the pre-polymerization solution. The photoabsorber additive material can be biocompatible. The photoabsorber additive material can be hydrophilic. The photoabsorber additive material can be hydrophobic.

In accordance with various embodiments, the photoabsorber additive material can be one of a food dye, tartrazine, sunset yellow FCF (Yellow No. 6), Brilliant Blue FCF (FD&C Blue No. 1), Indigo Carmine (FD&C Blue No. 2), Fast Green FCF (FD&C Green No. 3) anthocyanins, anthocyanidin, erythrosine (FD&C Red No.3), Allura Red AC (FD&C Red No. 40), riboflavin (Vitamin B2, E101, E101a, E106), ascorbic acid (Vitamin C), Quinoline Yellow WS, carmoisine (azorubine), Ponceau 4R (E124), Patent Blue V (E131), Green S (E142), Yellow 2G (E107), Orange GGN (E111), Red 2G (E128), caramel color, phenol red, Methyl orange, 4-nitrophenol, and NADH disodium salt.

In accordance with various embodiments, the hydrophobic photoabsorber can include a solvent. The solvent can be selected from the group consisting of dimethyl sulfoxide, ethanol (50-100%), isopropyl alcohol, (50-100%) dimethylformamide, N-Methyl-2-pyrrolidone, tetrahydrofuran, chloroform, methylene chloride, toluene, hexafluoro-2-propanol, and any combination thereof.

In accordance with various embodiments, the photoabsorber additive material can be one of Curcumin (E100), turmeric, alpha carotene, beta carotene, canthaxanthin (keto-carotenoid), cochineal extract, paprika, saffron, ergocalciferol (vitamin D2), cholecalciferol (vitamin D3), Citrus Red 2, annatto extract, and Lycopene.

In accordance with various embodiments, the apparatus can further comprise plant, bacterial, or mammalian cells. The apparatus can further comprise one or more of liver, lung, bone, endothelial, cardiac, pancreas, kidney, epithelial, muscle, cartilage, stem cells, skin, or eye cells.

Referring now to FIG. 18, a schematic illustration of an example apparatus 100 for fabricating a hydrogel construct is provided, in accordance with various embodiments. Apparatus 100 can include, for example, a frame 110, a stage 120, an electronics board 130, a container 140, a projector 160, an optional mirror 170, and a build platform 180. Stage 120 can be attached to frame 110 and can include a motor attached to frame 110. The motor can be a z-axis drive motor. Electronics board 130 can be configured for controlling movement of the stage with respect to the frame. Projector 160 can be configured for projecting images. The images can be image slices of a 3D model of the hydrogel matrix construct. Mirror 170 can be a 45° mirror. Mirror 170 can be configured for reflecting the images projected from projector 160 into the container 140. Note that optional mirror 170 can be excluded from the apparatus if the lens on the projector is already at an angle not necessitating a mirror, e.g., the projector projecting the image directly onto the desired surface. Build platform 180 can be configured for holding a substrate 190, wherein the hydrogel matrix construct can be formed on the substrate 190. Substrate 190 can be integrated into apparatus 100 or can be component wholly separate from the apparatus and, thus, can be considered to be not part of the apparatus.

The container 140 can be configured to receive a solution 150. As discussed above, solution 150 can be a pre-polymerization solution comprising a photosensitive polymer and a photoabsorber additive material suitable to control light penetration.

Referring back to the example apparatus 800 of FIG. 8B discussed above, apparatus 800 can include a motor 810 (which can be attached to a frame 840), controlled by an actuator 815, onto which the build platform 820 is attached. The back of the printer can house the motherboard 825 that controls the motor and performs input/output with any additional sensors or switches. The front of the printer contains an optional mirror 830 placed to reflect incident patterns from a projector 835 onto a dish containing the photosensitive materials. Note that optional mirror 830 can be excluded from the apparatus if the lens on the projector is already at an angle not necessitating a mirror, e.g., the projector projecting the image directly onto the desired surface

In accordance with various embodiments, the various apparatus embodiments can be incorporated as part of a system. An example system 200 for manufacturing a hydrogel matrix construct is illustrated in FIG. 19 and will discussed in greater detail below.

The system for manufacturing a hydrogel matrix construct can comprise a processor. The processor can include a modeling engine. The modeling engine can be configured to create a 3D model of the hydrogel matrix construct based on a tessellation of polyhedra having a number of faces connected by edges and vertices. The modeling engine can be configured to generate a first vascular component of the model. The modeling engine can be configured to generate a second vascular component of the model. The modeling engine can be configured to combine the first and second vascular components of the model.

The system can further comprise an apparatus configured for manufacturing the hydrogel matrix construct. The apparatus can comprise a frame, a stage with z-axis drive motor attached to the frame, an electronics board for controlling movement of the stage with respect to the frame, a container configured for holding a solution, a projector for projecting images, wherein the images are image slices of a 3D model of the hydrogel matrix construct, an optional 45° mirror for reflecting the images projected from the projector into the container, and a build platform configured for holding a substrate, wherein the hydrogel matrix construct is formed on the substrate. Note that the optional 45° mirror can be excluded from the apparatus if the lens on the projector is already at an angle not necessitating a mirror, e.g., the projector projecting the image directly onto the desired surface. These various components, in accordance with various embodiments, can all be provided as part of single apparatus. These various components, however, can also be provided in various other configurations whereby any one or more components can be provided as part of further sub-apparatus that interact with the main apparatus to together provide fundamentally the same functionality as those embodiments where all components are provided within the single apparatus. Further, various embodiments can include only a portion of the components provided above as it should not be assumed that each and every component recited is necessary for the proper functionality of an apparatus for manufacturing a hydrogel matrix construct.

In accordance with various embodiments, the solution can be a pre-polymerization solution comprising a photosensitive polymer and a photoabsorber additive material suitable to control light penetration. The additive material can be removable from a solid formed of the pre-polymerization solution. The photoabsorber additive material can be biocompatible. The photoabsorber additive material can be hydrophilic. The photoabsorber additive material can be hydrophobic.

In accordance with various embodiments, the hydrophobic photoabsorber additive material can include a solvent. The solvent can be selected from the group consisting of dimethyl sulfoxide, ethanol (50-100%), isopropyl alcohol, (50-100%) dimethylformamide, N-Methyl-2-pyrrolidone, tetrahydrofuran, chloroform, methylene chloride, toluene, hexafluoro-2-propanol, and any combination thereof.

In accordance with various embodiments, the solution can include a thickener to prevent cell settling. In accordance with various embodiments, the thickener includes xanthan gum having a concentrations about 0.02 wt% to about 2 wt%, about 0.03 wt% to about 1.5 wt%, about 0.04 wt% to about 1.0 wt%, or about 0.05 wt% to about 0.5 wt%, inclusive of any thickener values therebetween.

In accordance with various embodiments, the photoabsorber additive material can be one of a food dye, tartrazine, sunset yellow FCF (Yellow No. 6), Brilliant Blue FCF (FD&C Blue No. 1), Indigo Carmine (FD&C Blue No. 2), Fast Green FCF (FD&C Green No. 3) anthocyanins, anthocyanidin, erythrosine (FD&C Red No.3), Allura Red AC (FD&C Red No. 40), riboflavin (Vitamin B2, E101, E101a, E106), ascorbic acid (Vitamin C), Quinoline Yellow WS, carmoisine (azorubine), Ponceau 4R (E124), Patent Blue V (E131), Green S (E142), Yellow 2G (E107), Orange GGN (E111), Red 2G (E128), caramel color, phenol red, Methyl orange, 4-nitrophenol, and NADH disodium salt.

In accordance with various embodiments, the photoabsorber additive material can be one of Curcumin (E100), turmeric, alpha carotene, beta carotene, canthaxanthin (keto-carotenoid), cochineal extract, paprika, saffron, ergocalciferol (vitamin D2), cholecalciferol (vitamin D3), Citrus Red 2, annatto extract, and Lycopene.

In accordance with various embodiments, the apparatus can further comprise plant, bacterial, or mammalian cells. The apparatus can further comprise one or more of liver, lung, bone, endothelial, cardiac, pancreas, kidney, epithelial, muscle, cartilage, stem cells, skin, or eye cells.

In accordance with various embodiments, the generating the first vascular component of the model can comprises removing the faces and/or the vertices of the polyhedra, and using the remaining topology as a vascular skeleton. The generating the first vascular component of the model can further comprise skinning the skeleton with a polygonal mesh and then smoothing the result to produce cylindrical channels along the edges of the model with intervessel junctions located at each vertex location.

In accordance with various embodiments, the generating the second vascular component of the model can comprises scaling the faces of the 3D model along local face normals such that the airway is nested inside the vasculature component, forming independent fluidic connections to the vasculature and to the airway, and performing a Boolean subtraction from a solid volume.

In accordance with various embodiments, the processor can be further configured to fabricate a hydrogel alveolar construct. The processor can fabricate a hydrogel alveolar construct by fusing multiple spheres in a radially symmetric fashion to create an airway topology and offsetting the airway topology to generate a vascular surface topology. The fabrication of the hydrogel aveolar construct can further include tessellating, skeletonizing, and skinning the vascular surface topology to produce a Voronoi vascular topology. The tessellating includes performing a tessellation of polyhedral. The tessellation of polyhedra can be a Weaire-Phelan space-filling foam model of two dodecahedron and six tetrakaidecahedron cells. The fabrication of the hydrogel aveolar construct can further include performing a Boolean subtraction of the original airway and the tessellated Voronoi vascular topology from a solid volume to generate a final 3D model for additive manufacturing.

Referring now to FIG. 19, a schematic illustration of an example system 200 for fabricating a hydrogel construct is provided, in accordance with various embodiments. System 200 can comprise an apparatus and processor. The example apparatus illustrated from FIG. 19 is apparatus 100 illustrated in FIG. 18. Refer above for detailed discussion of apparatus 100 of FIG. 18. Processor 220 can include a modeling engine 250.

Processor 220 can be communicatively connected to apparatus 100. In various embodiments, processor 220 can be communicatively connected to apparatus 100 via a network connection that can be either a "hardwired" physical network connection (e.g., Internet, LAN, WAN, VPN, etc.) or a wireless network connection (e.g., Wi-Fi, WLAN, etc.). In various embodiments, processor 220 can be part of a workstation, mainframe computer, distributed computing node (part of a "cloud computing" or distributed networking system), personal computer, mobile device, etc.

In accordance with various embodiments, the processor 220 and apparatus 100 can be part of an integrated apparatus. As such, in FIG. 19, processor 220 can be provided within apparatus 100. Alternatively, processor 220 can be hosted by a different device than the apparatus 100. Moreover, processor 220 can be part of a distributed network system.

As discussed above, and in accordance with various embodiments, build platform 180 can be configured for holding a substrate 190, wherein the hydrogel matrix construct can be formed on the substrate 190. Substrate 190 can be integrated into apparatus 100 or can be component wholly separate from the apparatus and, thus, can be considered to be not part of the apparatus.

As discussed above, and in accordance with various embodiments, container 140 can be configured to receive a solution 150. As discussed above, solution 150 can be a pre-polymerization solution comprising a photosensitive polymer and a photoabsorber additive material suitable to control light penetration.

As discussed above, and in accordance with various embodiments, modeling engine 250 can be configured to create a 3D model of the hydrogel matrix construct based on a tessellation of polyhedra having a number of faces connected by edges and vertices. The modeling engine can be configured to generate a first vascular component of the model. The modeling engine can be configured to generate a second vascular component of the model. The modeling engine can be configured to combine the first and second vascular components of the model.

It should be understood that, in accordance with various embodiments, that modeling engine 250 can be configured to handle all the necessary steps accomplished within processor 220. This is what is illustrated by example processor 220 and associated modeling engine 250 in FIG. 19.

Further, in accordance with various embodiments, the functions and steps described in association with modeling engine 250 can be divided among any number of separate engines or modules within processor 220. For example, the steps of creating a 3D model, generating a first and second vascular component of the model, and combining the vascular components can each be accomplished by separate engines (e.g., creation engine, generation engine and combination engine) and/or by separate processors including one or more separate engines. Moreover, at least a portion of these steps can be combined into a single engine (e.g., a component engine that provides for the generation and combination steps).

FIG. 20 is a flowchart for an example method S100 of fabricating a 3D hydrogel construct, in accordance with various embodiments. As shown in FIG. 20, the method S100 includes creating a 3D model of the construct based on a tessellation of polyhedra having a number of faces connected by edges and vertices at step S110. The method S100 also includes generating a first vascular component of the model at step S120. In accordance with various embodiments herein, generating of the first vascular component of the model includes removing the faces and/or the vertices of the polyhedra, and using the remaining topology as a vascular skeleton, and skinning the skeleton with a polygonal mesh, and then smoothing the result to produce cylindrical channels along the edges of the model with intervessel junctions located at each vertex location.

The method S100 further includes generating a second vascular component of the model at step S130. In accordance with various embodiments, generating the second vascular component of the model includes scaling the faces of the 3D model along local face normals such that the second vascular component is nested inside the first vascular component, forming independent fluidic connections to the first vascular component and to the second vascular component, and performing a Boolean subtraction from a solid volume.

As shown in FIG. 20, the method S100 includes combining the first and second vascular components of the model at step S140.

In accordance with various embodiments, the method S100 optionally includes fabricating a hydrogel alveolar construct at step S150. In accordance with various embodiments, the fabrication of the hydrogel alveolar construct includes fusing multiple spheres in a radially symmetric fashion to create an airway topology, offsetting the airway topology to generate a vascular surface topology, tessellating, skeletonizing, and skinning the vascular surface topology to produce a Voronoi vascular topology, wherein tessellating comprises performing a tessellation of polyhedral, and performing a Boolean subtraction of the original airway and the tessellated Voronoi vascular topology from a solid volume to generate a final 3D model for additive manufacturing. In accordance with various embodiments, the tessellation of polyhedra is a Weaire-Phelan space-filling foam model of two dodecahedron and six tetrakaidecahedron cells.

FIG. 21 is a flowchart for an example method S200 of manufacturing a hydrogel matrix construct, in accordance with various embodiments. As shown in FIG. 21, the method S200 includes, at step 210, creating a 3D model of the hydrogel matrix construct using a design software. In accordance with various embodiments, the 3D model of the hydrogel matrix construct includes a first computational algorithm that yields a first tubular channel network in the hydrogel matrix construct, and a second computational algorithm, different from the first computational algorithm, that yields a second tubular channel network in the hydrogel matrix, wherein the first and second tubular channel networks are two independent, entangled vascular networks.

At step S220, the method S200 includes converting the 3D model to a format suitable for use in a 3D additive manufacturing software to yield a formatted 3D model to be generated using a 3D additive manufacturing machine. The method S200 further includes directing the additive manufacturing machine to generate the model at step S230.

According to the claimed invention , the computational algorithm is a tessellation of polyhedra.

As shown in FIG. 21, the method S200 optionally includes supplying a pre-polymerization solution to the 3D additive manufacturing machine wherein the pre-polymerization solution includes a photoabsorber at step S240. In accordance with various embodiments, the pre-polymerization solution includes one or more types of bacterial, mammalian, and plant cells.

The method S200 further optionally includes comprising lining the first tubular channel network or second tubular channel network with cells at step S250.

At step S260, the method S200 optionally includes embedding the hydrogel matrix with cells. In accordance with various embodiments, the cells are one or more of liver, lung, bone, endothelial, cardiac, pancreas, kidney, epithelial, muscle, cartilage, stem cells, skin, or eye cells.

The method S200 optionally includes comprising delivering heat while the 3D additive manufacturing machine generates the 3D model at step S270. In accordance with various embodiments, heat is added via a silicone heater, heat lamps, or infrared LEDs.

The method S200 optionally includes enclosing the 3D model in a heating enclosure during generation of the 3D model at step S280.

FIG. 22 is a flowchart for an example method S300 of fabricating a 3D hydrogel construct, according to various embodiments. As shown in FIG. 22, the method S300 includes using a computer-implemented process to create a 3D model of the construct based on a tessellation of polyhedra having a number of faces connected by edges and vertices at step S310. At step S320, the method S300 includes using the computer-implemented process to generate a first vascular component of the model. The method S300 further includes using the computer-implemented process to generate a second vascular component of the model at step S330. The method S300 includes using the computer-implemented process to combine the first and second vascular components of the model at step S340.

As shown in FIG. 22, the method S300 optionally includes using the computer-implemented process to generate the first vascular component of the model by removing the faces of the polyhedra, and using the remaining vertices and edges of the topology as a vascular skeleton at step S350.

The method S300 optionally includes, at step S360, using the computer-implemented process to generate the first vascular component of the model by skinning the skeleton with a polygonal mesh and then smoothing the result to produce cylindrical channels along the edges of the model with intervessel junctions located at each vertex location.

The method S300 optionally includes, at step S370, using a computer-implemented process to generate the second vascular component of the model by scaling the faces of the 3D model along local face normals such that the second vascular component is nested inside the first vascular component, forming independent fluidic connections to the first vascular component and to the second vascular component, and performing a Boolean subtraction from a solid volume.

As shown in FIG. 22, the method S300 optionally includes, at step S380, fabricating a hydrogel alveolar construct by fusing multiple spheres in a radially symmetric fashion to create an airway topology, offsetting the airway topology to generate a vascular surface topology, tessellating, skeletonizing, and skinning the vascular surface topology to produce a Voronoi vascular topology, wherein tessellating comprises performing a tessellation of polyhedral.

The method S300 optionally includes, at step S390, performing a Boolean subtraction of the original airway and the tessellated Voronoi vascular topology from a solid volume to generate a final 3D model for additive manufacturing. In accordance with various embodiments, the tessellation of polyhedra is a Weaire-Phelan space-filling foam model of two dodecahedron and six tetrakaidecahedron cells.

In accordance with various embodiments, a non-transitory computer-readable medium in which a program is stored for causing a computer to perform a method of fabricating a 3D hydrogel construct is provided. In accordance with various embodiments, the method includes creating a 3D model of the construct based on a tessellation of polyhedra having a number of faces connected by edges and vertices. In accordance with various embodiments, the method includes generating a first vascular component of the model, generating a second component of the model, and combining the first and second vascular components of the model. In accordance with various embodiments, the method includes generating the first vascular component of the model by removing the faces and/and or the vertices of the polyhedra, and using the remaining topology as a vascular skeleton. In accordance with various embodiments, the method includes generating the first vascular component of the model further comprises by skinning the skeleton with a polygonal mesh and then smoothing the result to produce cylindrical channels along the edges of the model with intervessel junctions located at each vertex location.

In accordance with various embodiments, the method includes generating the airway component of the model comprises by scaling the faces of the 3D model along local face normals such that the airway is nested inside the vasculature (vascular) component, forming independent fluidic connections to the vasculature and to the airway, and performing a Boolean subtraction from a solid volume. In accordance with various embodiments, the method includes fabricating a hydrogel alveolar construct by fusing multiple spheres in a radially symmetric fashion to create an airway topology, offsetting the airway topology to generate a vascular surface topology, tessellating, skeletonizing, and skinning the vascular surface topology to produce a Voronoi vascular topology, wherein tessellating comprises performing a tessellation of polyhedral, and performing a Boolean subtraction of the original airway and the tessellated Voronoi vascular topology from a solid volume to generate a final 3D model for additive manufacturing. In accordance with various embodiments, the tessellation of polyhedra is a Weaire-Phelan space-filling foam model of two dodecahedron and six tetrakaidecahedron cells.

FIG. 23 is a block diagram that illustrates a computer system 300, in accordance with various embodiments, that can be implemented to perform the various embodiments recited herein including, for example, various non-transitory computer-readable media in which a program is stored for causing a computer to perform various methods herein. In various embodiments, the computer system 300 can be a workstation, mainframe computer, distributed computing node (part of a "cloud computing" or distributed networking system), personal computer, mobile device, etc.

In accordance with various embodiments, the computer system 300 can include a bus 302 or other communication mechanism for communicating information, and a processor 304 coupled with bus 302 for processing information. In various embodiments, computer system 300 can also include a memory, which can be a random access memory (RAM) 306 or other dynamic storage device, coupled to bus 302 for determining instructions to be executed by processor 304. Memory also can be used for storing temporary variables or other intermediate information during execution of instructions to be executed by processor 304. In various embodiments, computer system 300 can further include a read only memory (ROM) 308 or other static storage device coupled to bus 302 for storing static information and instructions for processor 304. A storage device 310, such as a magnetic disk or optical disk, can be provided and coupled to bus 302 for storing information and instructions.

In various embodiments, computer system 300 can be coupled via bus 302 to a display 312, such as a cathode ray tube (CRT) or liquid crystal display (LCD), for displaying information to a computer user. An input device 314, including alphanumeric and other keys, can be coupled to bus 302 for communicating information and command selections to processor 304. Another type of user input device is a cursor control 316, such as a mouse, a trackball or cursor direction keys for communicating direction information and command selections to processor 304 and for controlling cursor movement on display 312. This input device 314 typically has two degrees of freedom in two axes, a first axis (i.e., x) and a second axis (i.e., y), that allows the device to specify positions in a plane. However, it should be understood that input devices 314 allowing for 3 dimensional (x, y and z) cursor movement are also contemplated herein.

Consistent with certain implementations of the present teachings, results can be provided by computer system 300 in response to processor 304 executing one or more sequences of one or more instructions contained in memory 306. Such instructions can be read into memory 306 from another computer-readable medium or computer-readable storage medium, such as storage device 310. Execution of the sequences of instructions contained in memory 306 can cause processor 304 to perform the processes described herein. Alternatively hard-wired circuitry can be used in place of or in combination with software instructions to implement the present teachings. Thus, implementations of the present teachings are not limited to any specific combination of hardware circuitry and software.

The term "computer-readable medium" (e.g., data store, data storage, etc.) or "computer-readable storage medium" as used herein refers to any media that participates in providing instructions to processor 304 for execution. Such a medium can take many forms, including but not limited to, non-volatile media, volatile media, and transmission media. Examples of non-volatile media can include, but are not limited to, optical, solid state, magnetic disks, such as storage device 310. Examples of volatile media can include, but are not limited to, dynamic memory, such as memory 306. Examples of transmission media can include, but are not limited to, coaxial cables, copper wire, and fiber optics, including the wires that comprise bus 302.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, or any other tangible medium from which a computer can read.

In addition to computer readable medium, instructions or data can be provided as signals on transmission media included in a communications apparatus or system to provide sequences of one or more instructions to processor 304 of computer system 300 for execution. For example, a communication apparatus may include a transceiver having signals indicative of instructions and data. The instructions and data are configured to cause one or more processors to implement the functions outlined in the disclosure herein. Representative examples of data communications transmission connections can include, but are not limited to, telephone modem connections, wide area networks (WAN), local area networks (LAN), infrared data connections, NFC connections, etc.

It should be appreciated that the methodologies described herein flow charts, diagrams and accompanying disclosure can be implemented using computer system 300 as a standalone device or on a distributed network of shared computer processing resources such as a cloud computing network.

The methodologies described herein may be implemented by various means depending upon the application. For example, these methodologies may be implemented in hardware, firmware, software, or any combination thereof. For a hardware implementation, the processing unit may be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, electronic devices, other electronic units designed to perform the functions described herein, or a combination thereof.

In various embodiments, the methods of the present teachings may be implemented as firmware and/or a software program and applications written in conventional programming languages such as C, C++, Python, etc. If implemented as firmware and/or software, the embodiments described herein can be implemented on a non-transitory computer-readable medium in which a program is stored for causing a computer to perform the methods described above. It should be understood that the various engines described herein can be provided on a computer system, such as computer system 300, whereby processor 304 would execute the analyses and determinations provided by these engines, subject to instructions provided by any one of, or a combination of, memory components 306/308/310 and user input provided via input device 314.

While this specification contains many specific implementation details, these should not be construed as limitations on the scope of what is claimed, but rather as descriptions of features that can be specific to particular implementations. Certain features that are described in this specification in the context of separate implementations can also be implemented, in combination, in a single implementation. Conversely, various features that are described in the context of a single implementation can also be implemented in multiple implementations, separately, or in any suitable sub-combination.

Particular implementations of the subject matter have been described. Other implementations, alterations, and permutations of the described implementations are within the scope of the following claims as will be apparent to those skilled in the art. While operations are depicted in the drawings or claims in a particular order, this should not be understood as requiring that such operations be performed in the particular order shown or in sequential order, or that all illustrated operations be performed (some operations can be considered optional), to achieve desirable results. In certain circumstances, multitasking or parallel processing (or a combination of multitasking and parallel processing) can be advantageous and performed as deemed appropriate.

Accordingly, the previously described example implementations do not define or constrain the present disclosure.

## Claims

1. A device, comprising:
a hydrogel matrix including a photoabsorber; and
a void architecture in the matrix, having a first vessel architecture and a second vessel architecture that are each tubular and branching;
wherein the first and second vessel architectures are fluidically independent from each other and wherein one or both of the first vessel architecture and second vessel architecture is formed from a model based on a tessellation of polyhedra.

2. The device of claim 1, wherein the tessellation of polyhedra is a Weaire-Phelan space-filling foam model of two dodecahedron and six tetrakaidekahedron cells.

3. The device of claim 1, wherein the device allows at least 90% of visible light incident on the device to pass therethrough and allow imaging of the device.

4. The device of claim 1, wherein the photoabsorber is degradable independent of any degradation of the remaining hydrogel matrix.

5. The device of claim 1, wherein the photoabsorber is degradable by chemical or physical processes.

6. The device of claim 1, wherein the void architecture is multi-vascular.

7. The device of claim 1, wherein the void architecture is multi-vascular and further comprises a functional valve, and/or a fluidic static mixer.

8. The device of claim 1, wherein the device is monolithic, and/or implantable.

9. The device of claim 1, wherein the hydrogel matrix is a photopolymerized hydrogel matrix , a porous hydrogel matrix, and/or biodegradable.

10. The device of claim 1, wherein the device is produced by additive manufacturing.

11. The device of claim 1, wherein the first vessel architecture and the second vessel architecture are entangled, or the first vessel architecture is ensheathed by the second vessel architecture.

12. The device of claim 1, wherein the photoabsorber is hydrophilic.

13. The device of claim 12, wherein the photoabsorber is one of a food dye, tartrazine, sunset yellow FCF (Yellow No. 6), Brilliant Blue FCF (FD&C Blue No. 1), Indigo Carmine (FD&C Blue No. 2), Fast Green FCF (FD&C Green No. 3) anthocyanins, anthocyanidin, erythrosine (FD&C Red No.3), Allura Red AC (FD&C Red No. 40), riboflavin (Vitamin B2, E101, E101a, E106), ascorbic acid (Vitamin C), Quinoline Yellow WS, carmoisine (azorubine), Ponceau 4R (E124), Patent Blue V (E131), Green S (E142), Yellow 2G (E107), Orange GGN (E111), Red 2G (E128), caramel color, phenol red, Methyl orange, 4-nitrophenol, and NADH disodium salt.

14. The device of claim 1, wherein the photoabsorber is hydrophobic.

15. The device of claim 14, wherein the photoabsorber is one of Curcumin (E100), turmeric, alpha carotene, beta carotene, canthaxanthin (keto-carotenoid), cochineal extract, paprika, saffron, ergocalciferol (vitamin D2), cholecalciferol (vitamin D3), Citrus Red 2, annatto extract, and Lycopene.

16. The device of claim 7, wherein the fluidic static mixer comprises photoabsorber, tartrazine, or fin elements with 180° twists.

17. The device of claim 1, further comprising a thickener to prevent cell settling, the thickener comprising xanthan gum having a concentration ranging from 0.02 wt% to 2 wt%.

## Patentansprüche

1. Eine Vorrichtung umfassend:
eine Hydrogelmatrix, die einen Photoabsorber enthält; und
eine Hohlraumarchitektur in der Matrix, die eine erste Gefäßarchitektur und eine zweite Gefäßarchitektur aufweist, die jeweils röhrenförmig und verzweigt sind;
wobei die erste und die zweite Gefäßarchitektur strömungstechnisch unabhängig voneinander sind und wobei die erste Gefäßarchitektur oder die zweite Gefäßarchitektur oder beide aus einem Modell gebildet sind, das auf einem Mosaik von Polyedern basiert.

2. Die Vorrichtung nach Anspruch 1, wobei das Mosaik aus Polyedern ein Weaire-Phelan-Raum-füllendes Schaummodell aus zwei Dodekaeder- und sechs Tetrakaidekaederzellen ist.

3. Die Vorrichtung nach Anspruch 1, wobei die Vorrichtung mindestens 90 % des auf die Vorrichtung einfallenden sichtbaren Lichts durchlässt und die Abbildung der Vorrichtung ermöglicht.

4. Die Vorrichtung nach Anspruch 1, wobei der Photoabsorber unabhängig von einem Abbau der restlichen Hydrogelmatrix abbaubar ist.

5. Die Vorrichtung nach Anspruch 1, wobei der Photoabsorber durch chemische oder physikalische Prozesse abbaubar ist.

6. Die Vorrichtung nach Anspruch 1, wobei die Hohlraumarchitektur multivaskulär ist.

7. Die Vorrichtung nach Anspruch 1, wobei die Hohlraumarchitektur multivaskulär ist und ferner ein funktionelles Ventil und/oder einen statischen Fluidikmischer umfasst.

8. Die Vorrichtung nach Anspruch 1, wobei die Vorrichtung monolithisch und/oder implantierbar ist.

9. Die Vorrichtung nach Anspruch 1, wobei die Hydrogelmatrix eine photopolymerisierte Hydrogelmatrix, eine poröse Hydrogelmatrix und/oder biologisch abbaubar ist.

10. Die Vorrichtung nach Anspruch 1, wobei die Vorrichtung durch additive Fertigung hergestellt ist.

11. Die Vorrichtung nach Anspruch 1, wobei die erste Gefäßarchitektur und die zweite Gefäßarchitektur miteinander verflochten sind oder die erste Gefäßarchitektur von der zweiten Gefäßarchitektur umhüllt ist.

12. Die Vorrichtung nach Anspruch 1, wobei der Photoabsorber hydrophil ist.

13. Die Vorrichtung nach Anspruch 12, wobei der Photoabsorber einer der folgenden Lebensmittelfarbstoffe ist, Tartrazin, Sunsetgelb FCF (Gelb Nr. 6), Brilliantblau FCF (FD&C Blue No. 1), Indigo Carmin (FD&C Blue No. 2), Fast Green FCF (FD&C Green No. 3), Anthocyane, Anthocyanidin, Erythrosin (FD&C Red No. 3 ), Allurarot AC (FD&C Rot Nr. 40), Riboflavin (Vitamin B2, E101, E101a, E106), Ascorbinsäure (Vitamin C), Chinolingelb WS, Karmesin (Azorubin), Ponceau 4R (E124), Patentblau V (E131), Grün S (E142), Gelb 2G (E107), Orange GGN (E111), Rot 2G (E128), Karamellfarbe, Phenolrot, Methylorange, 4-Nitrophenol und NADH-Dinatriumsalz.

14. Die Vorrichtung nach Anspruch 1, wobei der Photoabsorber hydrophob ist.

15. Die Vorrichtung nach Anspruch 14, wobei der Photoabsorber einer der folgenden ist: Curcumin (E100), Kurkuma, Alpha-Carotin, Beta-Carotin, Canthaxanthin (Keto-Carotinoid), Cochenille-Extrakt, Paprika, Safran, Ergocalciferol (Vitamin D2), Cholecalciferol (Vitamin D3), Citrus Rot 2, Annatto-Extrakt und Lycopin.

16. Die Vorrichtung nach Anspruch 7, wobei der statische Fluidikmischer Photoabsorber, Tartrazin oder Lamellenelemente mit 180°-Drehung umfasst.

17. Die Vorrichtung nach Anspruch 1, die ferner ein Verdickungsmittel umfasst, um das Absetzen von Zellen zu verhindern, wobei das Verdickungsmittel Xanthangummi mit einer Konzentration im Bereich von 0,02 Gew.-% bis 2 Gew.-% umfasst.

## Revendications

1. Dispositif, comprenant :
une matrice d'hydrogel comprenant un photoabsorbeur ; et
une architecture vide dans la matrice, ayant une première architecture de vaisseau et une deuxième architecture de vaisseau qui sont chacune tubulaire et ramifiée ;
dans lequel les première et deuxième architectures de vaisseau sont indépendantes fluidiquement l'une de l'autre et dans lequel une ou les deux parmi la première architecture de vaisseau et la deuxième architecture de vaisseau est formée à partir d'un modèle basé sur une mosaïque de polyèdre.

2. Dispositif selon la revendication 1, dans lequel la mosaïque de polyèdre est un modèle en mousse de remplissage d'espace de Weaire-Phelan de deux cellules de dodécaèdre et six cellules de tétrakaidécaèdre.

3. Dispositif selon la revendication 1, où le dispositif permet à au moins 90 % de la lumière visible incidente sur le dispositif de passer à travers celui-ci et permet l'imagerie du dispositif.

4. Dispositif selon la revendication 1, dans lequel le photoabsorbeur est dégradable indépendamment de toute dégradation de la matrice d'hydrogel restante.

5. Dispositif selon la revendication 1, dans lequel le photoabsorbeur est dégradable par des procédés chimiques ou physiques.

6. Dispositif selon la revendication 1, dans lequel l'architecture vide est multi-vasculaire.

7. Dispositif selon la revendication 1, dans lequel l'architecture vide est multi-vasculaire et comprend en outre une valve fonctionnelle, et/ou un mélangeur statique fluidique.

8. Dispositif selon la revendication 1, où le dispositif est monolithique et/ou implantable.

9. Dispositif selon la revendication 1, dans lequel la matrice d'hydrogel est une matrice d'hydrogel photopolymérisée, une matrice d'hydrogel poreuse et/ou biodégradable.

10. Dispositif selon la revendication 1, où le dispositif est produit par fabrication additive.

11. Dispositif selon la revendication 1, dans lequel la première architecture de vaisseau et la deuxième architecture de vaisseau sont enchevêtrées, ou la première architecture de vaisseau est engainée par la deuxième architecture de vaisseau.

12. Dispositif selon la revendication 1, dans lequel le photoabsorbeur est hydrophile.

13. Dispositif selon la revendication 12, dans lequel le photoabsorbeur est un élément parmi un colorant alimentaire, une tartrazine, le jaune orangé sunset FCF (jaune N°6), le bleu brillant FCF (FD&C bleu N°1), le carmin d'indigo (FD&C bleu N°2), le vert solide FCF (FD&C vert N°3), les anthocyanines, l'anthocyanidine, l'érythrosine (FD&C rouge N°3), le rouge allura AC (FD&C rouge N°40), la riboflavine (vitamine B2, E101, E101a, E106), l'acide ascorbique (vitamine C), le jaune de quinoléine WS, la carmoisine (azorubine), le Ponceau 4R (E124), le bleu patenté V (E131), le vert S (E142), le jaune 2G (E107), l'orange GGN (E111), le rouge 2G (E128), le colorant caramel, le rouge de phénol, le méthylorange, le 4-nitrophénol et le sel disodique NADH.

14. Dispositif selon la revendication 1, dans lequel le photoabsorbeur est hydrophobe.

15. Dispositif selon la revendication 14, dans lequel le photoabsorbeur est l'un parmi la curcumine (E100), le curcuma, l'alpha-carotène, le bêta-carotène, la canthaxanthine (céto-caroténoïde), l'extrait de cochenille, le paprika, le safran, l'ergocalciférol (vitamine D2), le cholécalciférol (vitamine D3), le rouge citrus n°2, l'extrait de roucou, et le lycopène.

16. Dispositif selon la revendication 7, dans lequel le mélangeur statique fluidique comprend un photoabsorbeur, de la tartrazine, ou des éléments d'ailettes avec des torsions à 180°.

17. Dispositif selon la revendication 1, comprenant en outre un épaississant pour éviter la sédimentation de la cellule, l'épaississant comprenant de la gomme xanthane ayant une concentration allant de 0,02 % en poids à 2 % en poids.
